# EUROPÄISCHE PATENTSCHRIFT

(11) **EP 1 257 353 B1**
(45) Veröffentlichungstag und Bekanntmachung des Hinweises auf die Patenterteilung: **03.11.2004**
(21) Anmeldenummer: 01919315.0
(22) Anmeldetag: 20.02.2001
(51) Int. Cl.: B01J 13/02, A61K 7/00, C11D 17/00, D06M 23/12

(54) **WASCH- ODER REINIGUNGSMITTEL MIT KOMPONENTEN IN FORM VON MIKRO- UND/ODER NANOKAPSELN**
WASHING OR CLEANING COMPOSITION HAVING COMPONENTS IN FORM MICROCAPSULES AND/OR NANOCAPSULES
COMPOSITION DE LAVAGE OU NETTOYAGE COMPRENANT DES COMPOSANTES SOUS FORME DE MICRO- ET/OU NANOCAPSULES

(30) Priorität: 23.02.2000 DE 10008306; 23.02.2000 DE 10008307; 23.02.2000 DE 10008305
(43) Veröffentlichungstag der Anmeldung: 20.11.2002
(73) Patentinhaber: Henkel Kommanditgesellschaft auf Aktien, 40589 Düsseldorf (DE)
(72) Erfinder: EISFELD, Wolf, 40597 Düsseldorf (DE); KRUPP, Ute, 45307 Essen (DE); LOSSACK, Annett, 40599 Düsseldorf (DE); SCHEIDGEN, Arndt, 45239 Essen (DE); BRAUN, Verena, 70176 Stuttgart (DE)
(86) Internationale Anmeldenummer: PCT/EP2001/001887
(87) Internationale Veröffentlichungsnummer: WO 2001/062376

(56) Entgegenhaltungen:
- EP-A- 0 771 566
- US-A- 5 518 856
- US-A- 5 753 264
- DATABASE WPI Section Ch, Week 199140 Derwent Publications Ltd., London, GB; Class A87, AN 1991-291888 XP002170527 & JP 03 193979 A (UNITIKA LTD), 23. August 1991 (1991-08-23)

## Beschreibung

Die vorliegende Erfindung betrifft Wasch- oder Reinigungsmittel in Form von Kombinationsprodukten, welche Komponenten für die Textilreinigung und Komponenten für die Textilpflege in Form von Mikro- oder Nanokäpseln,. deren Oberfläche kationische Ladungen aufweisen, enthalten.

Mikro- bzw. Nanokapseln sind Pulver bzw. Teilchen, die aus einem Kern und einem den Kern umgebenden Wandmaterial bestehen, worin der Kern ein fester, flüssiger oder gasförmiger Stoff ist, der von dem festen, in der Regel pelymeren, Wandmaterial umhüllt ist. Sie können massiv sein, d.h. aus einem einzigen Material bestehen. Mikrokapseln weisen im Durchschnitt einen Durchmesser von 1 bis 1000 µm auf, während der Durchmesser der Nanokapseln zwischen 1 und 1000 nm bzw. zur Abgrenzung zwischen Mikro- und Nanokapsel zwischen 1 und nahezu 1000 nm liegt.

Mikro- und Nanokapseln werden insbesondere bei Arzneimitteln eingesetzt, z.B. zur Überführung von flüssigen, insbesondere auch von flüchtigen Verbindungen, in feste, freifließende Pulver, aber auch zur Stabilitätserhöhung der Wirkstoffe, zur Retardierung von Wirkstoffen, zum organspezifischen Transport der Wirkstoffe, zur Geschmacksüberdeckung wie auch zur Vermeidung von Unverträglichkeiten mit anderen Wirk- und Hilfsstoffen. Ein weiteres Einsatzgebiet von Mikrokapseln ist die Herstellung von kohlefreien Reaktivdurchschreibpapieren.

Beispiele für Verfahren zur Herstellung von Mikro- und Nanokapseln und deren Anwendung findet man in der Patentliteratur. So wird in dem US-Patent US 5,518,856 ein Verfahren zur Herstellung positiv geladener Mikrokapseln offenbart. Dabei offenbart jene Schrift im wesentlichen mikroverkapselte Repellentien für Läuse, z. B. in Form präparierter Ohrringe, sowie mikroverkapselte UV-Protectoren und Vitamin-Präparate, allerdings ohne in irgendeiner Weise den Themenkomplex Wasch- und Reinigungsmittel zu tangieren. Weiteren Stand der Technik, gemäß Database WPI Section Ch, Week 1991140 Derwent Publications Ltd., Lohdon, GB, Class A87, AN 1991-291888 XP002170527 & JP 03 193979 AQ (Unitika Ltd; 23.08.1991). stellt die Parfümierung von Fasermaterial tierischen Ursprungs durch Anwendung einer Tränklösung dar. Diese Tränklösung enthält parfümhaltige Mikrokapseln kationischer Ladung, wobei das Kapselmaterial Gelatine ist. Dabei wird das Fasermaterial tierischen Ursprungs vor der Anwendung der Tränklösung oxidiert, um eine anionische Oberflächenladung zu erzeugen. Nach der darauffolgenden Tränkung des Fasermaterials mit der Tränklösung wird das getränkte Fasermaterial einer Wärmebehandlung bei Temperaturen von beispielsweise 50°C ausgesetzt und anschtießend mit einem Reduktionsmittel behandelt. Hinweise auf Wasch- oder Reinigungsmittel finden sich jedoch auch hier nicht.

Durch die Auswahl der Kapsel- oder Wandmaterialien, wie natürlichen oder synthetischen Polymeren, kann die Wandung dicht, permeabel oder semipermeabet gestaltet werden. Somit ergibt sich eine Fülle von Möglichkeiten, die eingekapselte Substanz gesteuert freizusetzen, z.B. durch Zerstören der Hülle oder durch Permeation oder auch durch chemische Reaktionen, die im Inneren der Kapseln ablaufen können.

Die Zerstörung des Kapselmaterials, d.h. der Wandung, kann mechanisch von außen erfolgen und auch durch Erhitzen über den Siedepunkt des Kemmaterials von innen. Ferner können die Inhaltsstoffe durch Auflösen, Schmelzen oder Verbrennen des Wandmaterials freigesetzt werden.

Die Freisetzung des Kemmaterials über semipermeable Kapselwände kann z.B. durch Erhöhung des osmotischen Drucks im Inneren der Kapsel erfolgen, oder, wenn die Kapselwand für das Kemmaterial durchlässig ist, so tritt es langsam durch die Kapsetwandung hindurch und wird freigesetzt. Eine weitere Möglichkeit besteht darin, daß die Kapselwandung ihre Eigenschaften durch Verändern der sie umgebenden Phase (Wechsel von Luft zu Wasser, Änderung des pH-Wertes, etc.) semipermeabel wird und die Freisetzung des Kernmaterials wie zuvor beschrieben erfolgen kann.

Pflegekomponenten und auch Duftstoffe, die in Wasch- oder Reinigungsmitteln sowie in kosmetischen Produkten zur Reinigung und/oder zur Pflege des menschlichen Körpers enthalten sind, werden in der Regel nach der entsprechenden Behandlung wieder ausgespült. Dies betrifft im Bereich der kosmetischen Produkte insbesondere die sogenannten Rinse-off-Produkte. Im allgemeinen existieren nur geringe Bindungskräfte zwischen der Substratoberfläche (Textilfaser, Haut, Haarfaser) und den Mikro- bzw. Nanokapseln.

Der vorliegenden Erfindung lag die Aufgabe zugrunde, Wasch- oder Reinigungsmittel bereitzustellen, die textilpflegende Komponenten mit spezifischer Substantivität gegenüber Substraten beinhalten.

Überraschenderweise wurde festgestellt, daß, wenn die Oberfläche von Kapseln der genannten Größenordnung kationische Ladungen aufweist, diese aufgrund der Ladungsverhältnisse eine sehr gute Substantivität gegenüber Substraten wie Textilien, Haut und Keratinfasern, wie Haaren und Wolle, aufweisen, so daß sie auch nach der Behandlung, d.h. nach der Textilwäsche, Körperpflege beziehungsweise Haarwäsche etc. auf dem entsprechenden Substrat verbleiben. Der Freisetzungsmechanismus kann dann durch Druck, wie Rubbeln etc., oder durch Hitze, wie durch das Wasch- oder Duschwasser, beim Kämmen der Haare, beim Haartrocknen z.B. durch den Fön und bei der Textilbehandlung z.B. durch das Bügeleisen oder im Wäschetrockner, erfolgen.

Gegenstand der vorliegenden Erfindung ist demgemäß ein Wasch- oder Reinigungsmittel, bei dem es sich um ein Kombinationsprodukt handelt, das Komponenten für die Textilreinigung und Komponenten für die Textilpflege in Form von Mikro- und/oder Nanokapseln, deren Oberfläche kationische Ladungen aufweist, enthält.

Die erfindungsgemäßen Kapseln weisen vorzugsweise eine Teilchengröße im Nanobereich auf, insbesondere von 10 nm bis nahezu 1000 nm. Damit haben sie eine solche Größe, daß sie vom Anwender kaum beziehungsweise gar nicht wahrgenommen werden. Die Kapseln eignen sich insbesondere zum Einsatz in Wasch- oder Reinigungsmitteln sowie in kosmetischen Produkten, zum Beispiel Hautpflegemitteln und Haarpflegemitteln.

Bevorzugte Mikrokapseln, die vor allem in Wasch- oder Reinigungsmitteln einschließlich der Textilnachbehandlungsmittel wie Weichspülern vorteilhafterweise eingesetzt werden, weisen Teilchengrößen im Bereich von 1 bis 60 µm, vorzugsweise 1.5 bis 40 µm und insbesondere von 2 bis 30 µm auf.

Mikrokapseln, insbesondere solche mit einer Teilchengröße von 100 bis 1000 µm, sind bereits mit bloßem Auge sichtbar. Sie werden vorzugsweise immer dann eingearbeitet werden, wenn den erfindungsgemäßen Mitteln ästhetische und optische Effekte verliehen werden sollen.

Auch Mischungen aus Mikro- und Nanokapseln, also im Bereich von 1 nm bis 1000 µm können eingesetzt werden. Insbesondere bevorzugt sind dabei solche, welche einen Teilchengrößenbereich von 10 nm bis 1000 µm abdecken.

Die Kapseln können aus einem Wand- und Kernmaterial aufgebaut sein, wobei das Kemmaterial eine verkapselte Substanz, z. B. einen Wirkstoff, enthält. Die Kapseln können aber auch massiv oder kompakt sein, wobei diese Begriffe im Rahmen der vorliegenden Erfindung mit gleicher Bedeutung angewendet werden, bzw. der Kern enthält keine aktive Komponente, ist also ein Hohlkörper, wobei in diesem Fall das Kapselmaterial (Wandmaterial) einen Aktivstoff enthält bzw. daraus besteht. In einer vorteilhaften Ausführungsform der Erfindung beträgt das Massenverhältnis Kem:Wand zwischen 99,9:0.1 und 2:1, vorzugsweise zwischen 95:5 und 70:30, insbesondere zwischen 90:10 und 75:25.

Enthalten die erfindungsgemäßen Kapseln freizusetzende Inhaltsstoffe. so erfolgt die Freisetzung dieser Inhaltsstoffe vorzugsweise mechanisch, z. B. durch Druck oder durch Temperaturerhöhung.

Werden die erfindungsgemäßen Kapseln in Textilwaschmitteln eingesetzt, so kann die Freisetzung durch Druck beim Tragen der Textilien oder auch, wenn die Textilien z.B. Handtücher etc. sind, beim Benutzen dieser erfolgen. Die Freisetzung durch Temperaturerhöhung kann beispielsweise im Wäschetrockner oder beim Bügeln geschehen, wo die Kapseln aufgeschmolzen werden beziehungsweise die verkapselten Substanzen einen entsprechend hohen Dampfdruck aufweisen und die Kapseln aufplatzen.

Es ist auch möglich, in das Wandmaterial photokatatytisch aktive Materialien einzuarbeiten, die durch Lichteinwirkung das Wandmaterial langsam zerstören, so daß die verkapselten Inhaltsstoffe freigesetzt werden.

Weitere Freisetzungsmechanismen sind pH-Sprung und elektrolytische Freisetzung.

Bei einer ggf. sprunghaften Änderung des pH-Werts der Umgebung der Kapseln erfolgt ein Auflösen des Kapselwandmaterials und die Freisetzung der Aktivsubstanz. Dieser Sprung geschieht z. B. beim Mischen von sauren oder alkalischen Wasch- bzw. Reinigungsmitteln in Wasser. So können z. B. mit der Wasch- bzw. Reinigungsmitteizusammensetzung unverträgliche Aktivsubstanzen abgeschirmt transportiert werden und in der Wasch-, Spül- oder Reinigungsflotte aktiviert werden und wirken.

Eine elektrolytische Freisetzung wird durch einen Sprung in der lonenkonzentration der Umgebung der Kapseln verursacht. Auch diese Änderung tritt z. B. beim Mischen einer Wasch- bzw. Reinigungsmittelzusammensetzung mit relativ hohem lonengehalt mit Wasser auf. Das Kapselwandmaterial löst sich bei diesem Sprung auf oder die Aktivsubstanz wird durch Osmose durch das (semipermeable bzw. gesprengte) Kapselwandmaterial freigesetzt.

Beim Einsatz der erfindungsgemäßen Kapseln in Hautpflegemitteln oder in Haarpflegemitteln kann die Freisetzung ebenfalls durch Druck, wie durch Reiben oder Rubbeln auf der Haut beziehungsweise auf dem Haar oder beim Kämmen, oder durch Erwärmen, z.B. durch die Temperatur des Duschwassers, beim Trocknen der Haare mittels Haartrockner, erfolgen.

In einer weiteren Ausführungsform ist das Wandmaterial der erfindungsgemäßen Kapseln semipermeabel, d.h. das Kemmaterial kann z.B. durch Wärme, also erhöhten Dampfdruck, durch das semipermeable Wandmaterial hindurchtreten und so freigesetzt werden.

Die positive Ladung auf der Oberfläche der Kapseln kann entweder im Kapselmaterial selbst begründet sein oder nachträglich aufgebracht werden. So kann das Kapselmaterial beispielsweise ganz oder teilweise aus einem kationischen Polymer oder einem entsprechend modifizierten Polymer bestehen. In einer weiteren Ausgestaltung der vorliegenden Erfindung kann die Kapsetoberfläche mit kationischen Verbindungen beschichtet werden. Die Beschichtung mit kationischen Verbindungen kann in einfacher Weise, z.B. durch Aufsprühen von Lösungen oder Suspensionen der Verbindungen oder Eintauchen der Kapseln in Lösungen oder Suspensionen dieser Verbindungen erfolgen.

Das Kapselmaterial, im folgenden auch Wandmaterial genannt, der erfindungsgemäßen Mikro- und/oder Nanokapseln kann ein beliebiges, zur Herstellung von derartigen Kapseln geeignetes Material sein, so z.B. aus natürlichen oder synthetischen Polymeren bestehen. Beispiele für derartige Polymere sind polymere Polysaccharide, wie Agarose oder Cellulose, Chitin, Chitosan, Proteine, wie Gelatine, Gummi arabicum, Ethylcellulose, Methylcellulose, Carboxymethylethylcellulose. Hydroxyethylcellulose, Celluloseacetate, Polyamide, Polycyanacrylate, Polylactide, Polyglycolide, Polyanilin, Polypyrrol, Polyvinylpyrrolidon, Polystyrol, Polyvinylalkohol, Copolymere aus Polystyrol und Maleinsäureanhydrid, Epoxidharze, Polyethylenimine, Copolymere aus Styrol und Methylmethacrylat, Polyacrylate und Polymethacrylate, Polycarbonate, Polyester, Silikone, Gemische aus Gelatine und Wasserglas, Gelatine und Polyphosphat, Celluloseacetat und Phthalat, Gelatine und Copolymeren aus Maleinsäureanhydrid und Methylvinylether, Celluloseacetatbutyrat sowie beliebige Gemische der voranstehenden, die kationische Gruppen aufweisen können.

Ebenso ist es möglich, daß das Wandmaterial aus Stärke oder Stärkederivaten besteht. Diese sind besonders bevorzugt, wenn die erfindungsgemäßen Kapseln im Mikround/oder Nanogrößenbereich in Waschmitteln eingesetzt werden. Bei einer dem Waschgang nachgeschalteten thermischen Kapselöffnung, beispielsweise beim Bügeln, können sie zur Appretur bzw. der Härtung von Textilien beitragen.

Das Wandmaterial kann gegebenenfalls vemetzt sein. Übliche Vemetzer sind Glutaraldehyd, Hamstoff/Pormatdehydharze, Tanninverbindungen, wie Tanninsäure, und deren Gemische.

Das Wandmaterial sollte eine solche Festigkeit und thermische Stabilität aufweisen, daß die Kapsel unter Lagerbedingungen nicht zerstört wird, aber eine mechanische Freisetzung der verkapselten Substanzen unter leichter Druckeinwirkung oder eine thermische Freisetzung bei Temperaturen von 35 bis 220°C ermöglicht wird.

Die verkapselten Substanzen, im folgenden auch Kernmaterial genannt, können aus beliebigen, festen, flüssigen oder gasförmigen Materialien bestehen, die in verkapselter Form in entsprechende Produkte eingearbeitet werden sollen. Vorzugsweise werden als Kemmaterialien Duftstoffe, wie Parfümöle, oder bei dem jeweiligen Einsatzgebiet pflegend wirkende Substanzen verwendet, wie Weichspüler, Appreturmittel, usw. Es können auch Textilhilfsmittel, wie Komponenten zur Textilausrüstung und -veredelung, wie Imprägniermittel, Appretur (z. B. Stärkederivate zum Härten von Textilien), Avivagemittel, Komponenten für die Pflegeleichtausrüstung, Griffvariatoren und Soil-Release-Ausrüstung, (z. B. Terephthalate, Fluorverbindungen), Antistatika, antimikrobielle und fungizide Mittel (z. B. quartäre Ammoniumverbindungen bzw. als antimikrobiell bzw. fungizid bekannte Alkohole, Säuren etc.), Geruchsabsorber (wie Zinkricinoleat oder ähnliche bekannte Verbindungen, Cyclodextrine etc.), Bügelhilfsmittel (wie Siliconöle, Siliconquats, Polymere wie Polyethylen etc.), hautpflegende Substanzen (natürliche Substanzen oder der Extrakte zur Hautpflege, Rückfetter, Hautschutz etc.), Vorbehandlungsmittel für die nächste Wäsche, die als Depot in den Kapseln enthalten sind, Knitterschutzmittel usw. als Kemmaterial eingesetzt werden. Hierbei ist eine Freisetzung der Wirkstoffe nach dem Spülgang bevorzugt.

Des Weiteren können auch Substanzen, deren direkter Kontakt mit dem Wasch- oder Reinigungsmittel bei der Lagerung unerwünscht ist, die aber bei der Anwendung (z. B. im Wasch-/Spülgang) freigesetzt werden sollen, im Kern der Kapseln enthalten sein. Hierzu zählen z. B. Bleichmittel, Bleichaktivatoren, Enzyme, Schauminhibitoren, anorganische Salze (z. B. als Gelbrecher zur verbesserten Einspülbarkeit von hochviskosen oder gelförmigen Flüssigformutierungen), Lösungsmittel, Farbauffhscher, Farbstoffe/Pigmente/Pertglanz, Soil-release-Verbindungen, optische Aufheller, Vergrauungsinhibitoren, Knitterschutzmittel, Farbübertragungsinhibitoren, antimikrobielle Wirkstoffe, Germizide, Fungizide, Antioxidantien, Korrosionsinhibitoren, Antistatika, Bügelhilfsmittel, Phobier- und Imprägniermittel, Quell- und Schiebefestmittel, UV-Absorber.

Für eine genauere Beschreibung der einzelnen Wirkstoffe wird auch auf die Beschreibung weiter unten (Inhaltsstoffe von Wasch- oder Reinigungsmitteln) verwiesen.

Als Parfümöle bzw. Duftstoffe können einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzyl-carbinylacetat, Phenylethylacetat, Linalylbenzoat, Benzylformiat, Ethyfmethylphenylgiycinat, Allylcyclohexylpropionat, Styrallylpropionat und Benzylsalicylat. Zu den Ethem zählen beispielsweise Benzylethylether, zu den Aldehyden z.B. die linearen Alkanale mit 8-18 C-Atomen. Citral (Geranial), Citronellal, Citronellyloxyacetaldehyd, Cyclamenaldehyd, Hydroxycitronellal, Lilial und Bourgeonal. Zu den Ketonen z.B. die Jonone, α-Isomethylionon und Methyl-cedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und α-Pinen. Als Duftstoff kann auch Eucalyptol (1,8-Cineol) eingesetzt werden. Bevorzugt werden jedoch Mischungen verschiedener Riechstoffe verwendet, die gemeinsam eine ansprechende Duftnote erzeugen. Solche Parfümöle können auch natürliche Riechstoffgemische enthalten, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskatellersalbeiöl, Kamillenöl, Nelkenöl, Melissenöl, Minzöl, Eukalyptusöl, Zimtblätteröl, Lindenblütenöl, Wacholderbeeröl, Vetiveröl, Olibanumöl, Galbanumöl und Labdanumöl sowie Orangenbtütenöl, Neroliol, Orangenschalenöl und Sandelhotzöl.

Außerdem können als Riechstoffe Nitrile, Sulfide, Oxime. Acetale, Ketale, Säuren, Schiffsche Basen, heterocyclische Stickstoffverbindungen wie Indol und Chinolin, Pyrazine, Amine wie Anthanilate, Amide, halogenorganische Verbindungen wie Roseacetat, nitrierte Verbindungen wie Nitromoschus, heterocyclische Schwefelverbindungen wie Thiazole und heterocylische Sauerstoffverbindungen wie Epoxide, die alle dem Fachmann als mögliche Riechstoffe bekannt sind, eingesetzt werden.

Es kann vorteilhaft sein, die Duftstoffe auf Träger aufzubringen, die die Haftung des Parfüms auf der Wäsche verstärken und durch eine langsamere Duftfreisetzung für langanhaltenden Duft der Textilien sorgen. Als solche Trägermaterialien haben sich beispielsweise Cyclodextrine bewährt. Dabei können die Cyclodextrin-Parfüm-Komplexe zusätzlich noch mit weiteren Hilfsstoffen beschichtet werden.

Im Hinblick auf die Stabilität der Kapseln hat sich sich als vorteilhaft erwiesen, relativ unpolare Parfümöle einzusetzen. Bevorzugt sind daher in einer Ausführungsform der Erfindung Parfümöle, die einen C log P-Wert von größer als 2, insbesondere von größer als 3 aufweisen.

Beispiele für pflegende Komponenten sind Vitamine und Provitamine, wie Vitamin A, Vitamin C, Vitamin E (α-Tocopherol), Vitamin F (Polyen-Fettsäuren), Panthenol (Provitamin B5), Betakarotin (Provitamin A) und deren Derivate (z. B. Ester wie Stearylascorbat), Pflanzenextrakte, Biopolymere, Antischuppenmittel, UV-Schutzmittel, Emollients (kosmetische Öle), Silikonöle, Conditioner, Glycerin, Polymere für Festigungseffekte beim Haar, kationische Polymere, Komponenten zur Textilausrüstung und -veredelung, wie Imprägniermittel, Appretur, Avivagemittet, Komponenten für die Pflegeleichtausrüstung, Griffvariatoren und Soit-Release-Ausrüstung, Antistatika, antimikrobielle und fungizide Mittel usw. und beliebige weitere Komponenten, die eine pflegende Wirkung auf Textilien, Haut und Haare haben und sich in Kapseln einarbeiten lassen. Im Falle von kosmetischen Anwendungen sind als pflegende Komponenten Tocopherole und deren lipidlösliche Derivate bevorzugt. Geeignete Tocopherole sind z.B. die natürlichen Tocopherole und deren Gemische sowie synthetische Tocopherole. Geeignete Derivate sind z.B. Tocopherolacetat, Tocopherolnicotinat, Tocopherolascorbat, Tocopheryfretinoat, Tocopherytsuccinat, Tocopherylünofeat oder Tocopherylbenzoat.

Die Herstellung der Kapseln kann in an sich bekannter Weise erfolgen, wie durch Phasentrennverfahren, mechanisch-physikalische Verfahren oder Polymerisationsverfahren, wie Suspensions- und Emulsionspolymerisation, Inverse Suspensionspolymerisation, Micellenpolymerisation, Grenzflächen-Polymerisationsverfahren, Grenzflächen-Ablagerung, in-situ-Polymerisation, Verdampfung von Lösungsmitteln aus Emulsionen, Suspensionsvemetzung, Bildung von Hydrogelen, Vernetzung in Lösung/Suspension, Systeme von Liposomen und in molekularem Maßstab, wobei Phasentrennverfahren, auch Koazervation genannt, besonders bevorzugt ist.

Koazervation bedeutet, daß ein gelöstes Polymer in eine polymerreiche, noch lösungsmittelhaltige Phase mittels Desolvatation, z. B. durch pH-Änderung, Temperaturänderung, Aussalzen, Änderung der Ionenstärke, Zusatz von Komplexbildnem (Komplexkoazervation), Zusatz von Nichtlösungsmitteln, überführt wird. Das Koazervat lagert sich an der Grenzfläche des zu verkapselnden Materials unter Ausbildung einer zusammenhängenden Kapselwand an und wird durch Trocknung oder Polymerisation verfestigt.

Physikalische Verfahren zur Herstellung der erfindungsgemäßen Mikro- bzw. Nanokapseln sind Sprühtrocknung, Wirbelschichtverfahren, oder Extrusionsverfahren (Coextrusion), Schmelzvertropfung bzw. Verprillung (Brace-Verfahren), Sprühgefriertrocknung.

In den genannten Grenzflächen-Polymerisationsverfahren erfolgt die Wandbildung durch Polykondensation oder Polyaddition aus monomeren oder oligomeren Ausgangsstoffen an der Grenzfläche einer Wasser/Öi-Emulsion oder Öl/Wasser-Emulsion.

Sofern das Wandmaterial der Kapseln nicht bereits kationische Ladungen aufweist, werden die Kapseln mit einer kationischen Verbindung beziehungsweise einem kationischen Polymer beschichtet. Dazu werden sie entweder mit einer Lösung oder Suspension dieser Verbindung besprüht oder darin getaucht. Auch hier kann z. B. die Sprühtrocknung eingesetzt werden.

Die kationischen Verbindungen, mit denen die erfindungsgemäßen Kapseln beschichtet sein können, können aus beliebigen kationischen Verbindungen ausgewählt werden, sofern sie eine entsprechende Substantivität gegenüber dem zu behandelnden Substrat aufweisen. Beispiele für solche Verbindungen sind quaternäre Ammoniumverbindungen, kationische Polymere und Emulgatoren, wie sie in Haarpflegemitteln und auch in Mitteln zur Textilavivage eingesetzt werden.

Beispiele für solche Verbindungen sind quartäre Ammoniumverbindungen, kationische Polymere und Emulgatoren, wie sie in Haarpflegemitteln und auch in Mitteln zur Textilavivage eingesetzt werden.

Geeignete Beispiele sind quartäre Ammoniumverbindungen der Formeln (I) und (II), 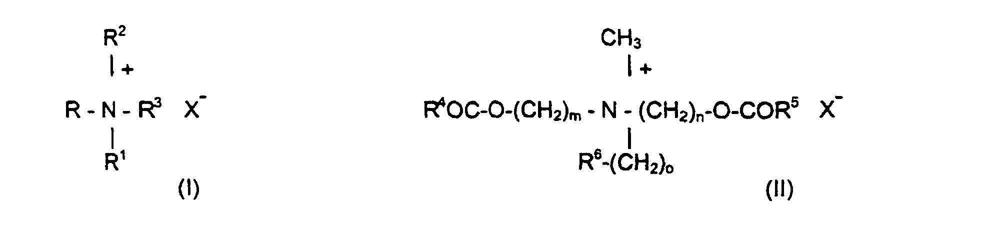 wobei R und R¹ für einen acyclischen Alkylrest mit 12 bis 24 Kohlenstoffatomen, R² für einen gesättigten C₁-C₄ Alkyl- oder -Hydroxyalkylrest steht, R³entweder gleich R, R¹ oder R² ist und COR⁴ und COR⁵ jeweils für einen aliphatischen Acylrest mit 12 bis 22 Kohlenstoffatomen mit 0, 1, 2 oder 3 Doppelbindungen steht sowie R⁶ für H oder OH steht, wobei m, n und o jeweils unabhängig voneinander den Wert 1, 2 oder 3 haben können und X entweder ein Halogenid-, Methosulfat- , Methophosphat- und/oder Phosphation ist, sowie Mischungen dieser Verbindungen. Besonders bevorzugt sind Verbindungen, die Alkylreste mit 16 bis 18 Kohlenstoffatomen enthalten Beispiele für Verbindungen der Formel (I) sind Didecyldimethylammoniumchlorid, Ditalgdimethylammoniumchlorid oder Dihexadecylammoniumchlorid. Beispiele für Verbindungen der Formel (II) sind Methyl-N-(2-hydroxyethyl)-N,N-di(talgacyl-oxyethyl)ammonium-methosulfat, Bis-(palmitoyl)-ethylhydroxyethyl-methyl-ammonium-methosulfat oder Methyl-N,N-bis(acyloxyethyl)-N-(2-hydroxyethyl)ammonium-methosulfat. Werden quartemierte Verbindungen der Formel (II) eingesetzt, die ungesättigte Alkylketten aufweisen, sind die Acylgruppen bevorzugt, deren korrespondierenden Fettsäuren eine Jodzahl zwischen 5 und 80, vorzugsweise zwischen 10 und 60 und insbesondere zwischen 15 und 45 aufweisen und die ein cis/trans-Isomerenverhältnis (in Gew.-%) von größer als 30 : 70, vorzugsweise größer als 50 : 50 und insbesondere größer als 70 : 30 haben. Handelsübliche Beispiele sind die von Stepan unter dem Warenzeichen Stepantex® vertriebenen Methylhydroxyalkyldialkoyloxyalkylammoniummethosulfate oder die unter Dehyquart® bekannten Produkte von Cognis bzw. die unter Rewoquat® bekannten Produkte von Goldschmidt-Witco.

Weitere bevorzugte Verbindungen sind die Diesterquats der Formel (III), die unter dem Namen Rewoquat® W 222 LM bzw. CR 3099 erhältlich sind und neben der Weichheit auch für Stabilität und Farbschutz sorgen. 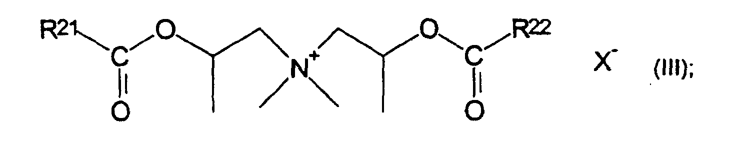 R²¹ und R²² stehen dabei unabhängig voneinander jeweils für einen aliphatischen Acylrest mit 12 bis 22 Kohlenstoffatomen mit 0,1, 2 oder 3 Doppelbindungen.

Neben den oben beschriebenen quartären Verbindungen können auch andere bekannte Verbindungen eingesetzt werden, wie beispielsweise quartäre imidazoliniumverbindungen der Formel (IV), 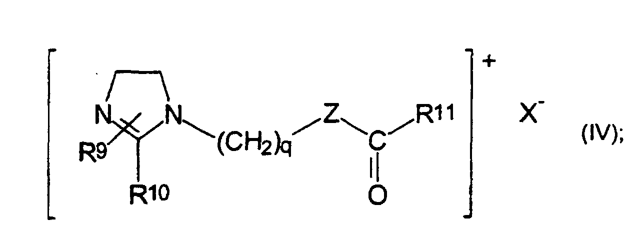 wobei R⁹ für H oder einen gesättigten Alkylrest mit 1 bis 4 Kohlenstoffatomen. R¹⁰ und R¹¹ unabhängig voneinander jeweils für einen aliphatischen, gesättigten oder ungesättigten Alkylrest mit 12 bis 18 Kohlenstoffatomen. R¹⁰ alternativ auch für O(CO)R²⁰ stehen kann, wobei R²⁰ einen aliphatischen, gesättigten oder ungesättigten Alkylrest mit 12 bis 18 Kohlenstoffatomen bedeutet, und Z eine NH-Gruppe oder Sauerstoff bedeutet und X⁻ ein Anion ist. q kann ganzzahlige Werte zwischen 1 und 4 annehmen.

Weitere geeignete quartäre Verbindungen sind durch Formel (V) beschrieben, 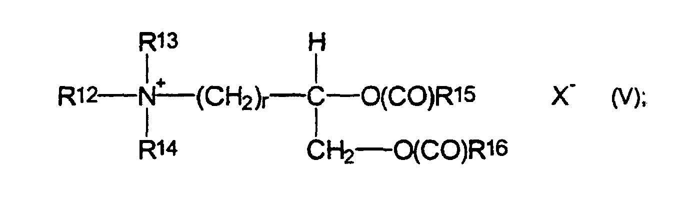 wobei R¹², R¹³ und R¹⁴ unabhängig voneinander für eine C₁₋₄ Alkyl-, Alkenyl- oder Hydroxyalkylgruppe steht, R¹⁵ und R¹⁶ jeweils unabhängig ausgewählt eine C₈₋₂₈-Alkylgruppe darstellt und r eine Zahl zwischen 0 und 5 ist.

Neben den Verbindungen der Formeln (I) und (II) können auch kurzkettige, wasserlösliche, quartäre Ammoniumverbindungen eingesetzt werden, wie Trihydroxyethylmethylammonium-methosulfat oder die Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid.

Auch protonierte Alkylaminverbindungen, die weichmachende Wirkung aufweisen, sowie die nicht quaternierten, protonierten Vorstufen der kationischen Emulgatoren sind geeignet.

Weitere erfindungsgemäß verwendbare kationische Verbindungen stellen die quatemisierten Proteinhydrolysate dar. Beispiele für Handelsprodukte stellen quatemisiertes Weizenprotein-hydrolysat Gluadin^{(R)} WQ, Gluadin^{(R)} WQT (Hersteller: Cognis Deutschland GmbH) dar.

Zu den geeigneten kationischen Polymeren zählen die Polyquaternium-Polymere, wie sie im CTFA Cosmetic Ingredient Dictionary (The Cosmetic, Toiletry und Fragrance, Inc., 1997) beschrieben werden. Beispiele sind insbesondere kationische Cellulosederivate, die auch als Merquats bezeichneten Polymere Polyquatemium-6, Polyquatemium-7; Polyquatemium-10-Polymere (Ucare Polymer JR 400; Amerchol;), Polyquatemium-4-Copolymere, wie Pfropfcopolymere mit einen Cellulosegerüst und quaternären Ammoniumgruppen, die über Allyldimethylammoniumchlorid gebunden sind, kationische Guarderivate, wie Guar-hydroxypropyltriammoniumchlorid, z. B. Cosmedia Guar (Hersteller: Cognis Deutschland GmbH), kationische quaternäre Zuckerderivate, z. B. das Handelsprodukt Glucquat® 100, gemäß CTFA-Nomenklatur ein "Lauryl Methyl Gluceth-10 Hydroxypropyl Dimonium Chloride", kationische Alkylpolyglucoside (APG-Derivate), kationische Polyacrylate, wie Eudragit^{(R)} RL 30 D (Hersteller: Röhm), Copolymere von PVP und Dimethylaminomethacrylat, Copolymere von Vinylimidazol und Vinylpyrrolidon, Aminosilicon-polymere und -copolymere.

Ebenfalls einsetzbar sind polyquatemierte Polymere (z. B. Luviquat Care von BASF) und auch kationische Biopolymere auf Chitinbasis und deren Derivate, beispielsweise das unter der Handelsbezeichnung Hydagen DCMF, CMFP, HCMG (Hersteller: Cognis Deutschland GmbH) erhältliche Chitosan sowie Chitosan-Derivate.

Erfindungsgemäß ebenfalls geeignet sind kationische Silikonöle wie beispielsweise die im Handel erhältlichen Produkte Q2-7224 (Hersteller: Dow Coming; ein stabilisiertes Trimethylsilylamodimethicon), Dow Coming 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) Abil® -Quat 3270 und 3272 (Hersteller: Goldschmidt-Rewo; diquartäre Polydimethyisiloxane, Quatemium-80), sowie Siliconquat Rewoquat® SQ 1 (Tegopren® 6922, Hersteller: Goldschmidt-Rewo).

Ebenfalls einsetzbar sind Verbindungen der Formel (VI), 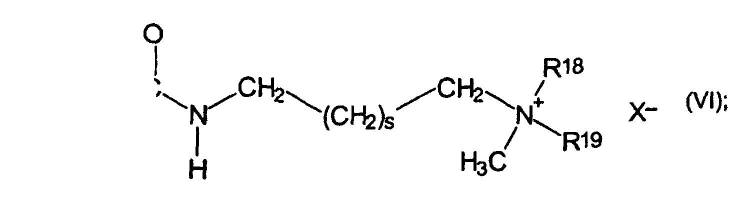 die Alkylamidoamine in ihrer nicht quaternierten oder, wie dargestellt, ihrer quaternierten Form, sein können. R¹⁷ kann ein aliphatischer Acylrest mit 12 bis 22 Kohlenstoffatomen mit 0, 1, 2 oder 3 Doppelbindungen sein. s kann Werte zwischen 0 und 5 annehmen. R¹⁸ und R¹⁹ stehen unabhängig voneinander jeweils für H, C₁₋₄-Alkyl oder Hydroxyalkyl. Bevorzugte Verbindungen sind Fettsäureamidoamine wie das unter der Bezeichnung Tego Amid® S 18 erhältliche Stearylamidopropyldimethylamin oder das unter der Bezeichnung Stepantex® X 9124 erhältliche 3-Talgamidopropyl-trimethylammonium-methosulfat, die sich neben einer guten konditionierenden Wirkung auch durch farbübertragungsinhibierende Wirkung sowie speziell durch ihre gute biologische Abbaubarkeit auszeichnen.

Ebenfalls einsetzbar sind Alkylamidoamine, insbesondere Fettsäureamidoamine wie das unter der Bezeichnung Tego Amid® S 18 erhältliche Stearylamidopropyldimethylamin, die sich neben einer guten konditionierenden Wirkung speziell durch ihre gute biologische Abbaubarkeit auszeichnen.

Die Mikro- und/oder Nanokapseln können sowohl in Wasch- oder Reinigungsmitteln oder in Mitteln für die Textilavivage oder auch in Mitteln zur Behandlung von Haut und Haaren, insbesondere in kosmetischen Mitteln zur Pflege und/oder zur Reinigung von Haut und Haaren, eingesetzt werden.

In einer möglichen Ausführungsform der vorliegenden Erfindung werden die Wasch- oder Reinigungsmittel zum Reinigen von harten Oberflächen einschließlich zur Geschirrreinigung eingesetzt. Mittel zur Reinigung von harten Oberflächen sind in der Regel nüssig bis gelförmig, während Mittel zur maschinellen Reinigung von Geschirr vorzugsweise pulverförmig sind oder in Tablettenform vorliegen.

Im Rahmen der vorliegenden Erfindung werden die Wasch- oder Reinigungsmittel vorzugsweise zur Textilbehandlung eingesetzt. Beispiele für Mittel zur Textilbehandlung sind Textilwaschmittel, wie Universalwaschmittel und Feinwaschmittel, Textilvörbehandlungsmittel und Fleckenbehandlungsmittel, sowie Nachbehandlungsmittel, wie Weichspüler. In einer weiteren Ausgestaltung sind die Mittel zur Textilbehandlung Kombinationsprodukte, die gleichzeitig die Komponenten für die Textilreinigung und Textilpflege enthalten, z. B. ein Universal- oder Feinwaschmittel, das Komponenten für die Textilpflege in Form von Mikro- und/oder Nanokapseln enthält. In einer weiteren Ausführungsform der Erfindung werden die erfindungsgemäßen Wasch- oder Reinigungsmittel als Textilreinigungsmittel zur Reinigung von Polstern oder Teppichen eingesetzt. Die Mittel zur Textilbehandlung können, in Abhängigkeit von ihrem Anwendungszweck, in flüssiger bis gelförmiger oder auch fester Form, als Pulver, Granulate oder Kompaktate, vorliegen.

Ein Mittel zur Pflege und Reinigung der Haut, das übliche in solchen Mitteln enthaltene Komponenten enthält und sich dadurch auszeichnet, dass ferner Mikro- und/oder Nanokapseln enthalten sind, deren Oberfläche kationische Ladungen aufweisen, ist ebenfalls möglich.

Als Beispiele für Mittel zur Pflege und Reinigung der Haut sind flüssige und stückförmige Wasch-, Dusch- und Badepräparate, Körpercremes und Lotionen, Bräunungsmittel, Gesichtscremes, Augenpilegemittel, Gesichtswasser und auch die Produkte der dekorativen Kosmetik, wie Lippenstifte und Lip-Gloss, Make-up und Gesichtspuder, Wimperntusche, Eyeliner, Kajalstifte, Lidschattenpräparate, Nagelpflegemittel, usw. zu nennen. Weitere Beispiele sind sogenannte Kombinationsprodukte, die gleichzeitig eine reinigende und eine pflegenden Wirkung auf die Haut haben, z. B. Hautreinigungsmittel, die Mikround/oder Nanokapseln mit Wirkstoffen mit rückfettenden und/oder pflegenden Eigenschaften als Kernmaterial enthalten.

Ein Mittel zur Reinigung und Pflege von Haaren, enthaltend übliche Inhattsstoffe, das dadurch gekennzeichnet ist, das es Nanokapseln enthält, deren Oberfläche kationische Ladungen aufweist, ist ebenfalls möglich.

Beispiele für Mittel zur Reinigung und Pflege von Haaren sind Haarshampoös, Haarpflegemittel wie Frisiercremes, -lotionen und gele, Haarfestiger, Haarsprays, Haarpomade, Haarspülungen und Kurpackungen, Haarverformungsmittel, Haarfärbemittel und Blondiermittel. Weitere Beispiele sind sogenannte Kombinationsprodukte, die gleichzeitig eine reinigende und eine pflegenden Wirkung auf die Haare haben, z. B. Haarshampoos, die Mikro- und/oder Nanokapseln mit pflegenden Substanzen als Kapselmaterial enthalten.

Die erfindungsgemäßen Mittel können in flüssiger bis gelförmiger oder auch in fester Form vorliegen und in beliebiger, für den jeweiligen Anwendungszweck vorteilhaften Form konfektioniert werden.

Liegen die Mittel in flüssiger bis gelförmiger Form vor, so handelt es sich in der Regel um wäßrige Zubereitungen, die ggf. noch weitere, mit Wasser mischbare organische Lösungsmittel sowie Verdickungsmittel enthalten. Zu den mit Wasser mischbaren organischen Lösungsmitteln zählen z. B. Hydrophilierungsmittel. Die Herstellung von flüssigen bis gelförmigen Zubereitungen kann kontinuierlich oder batchweise durch einfaches Vermischen der Bestandteile, ggf. bei erhöhter Temperatur erfolgen.

Aber auch sogenannte nicht-wäßrige Flüssigwaschmittel und nicht-wäßrige Gele zum Waschen von Textilien oder zur Reinigung von Geschirr sind denkbar. Ihr Wassergehalt liegt üblicherweise zwischen 0 und 10 Gew.-% und insbesondere nicht oberhalb von 5 Gew.-%.

Beispiele für Hydrophilierungsmittel sind ein- oder mehrwertigen Alkohole, Alkanolamine oder Glycolether, sofern sie mit Wasser mischbar sind. Vorzugsweise werden die Hydrophilierungsmittel ausgewählt aus Ethanol, n-oder i-Propanol, Butanolen, Ethylenglykolmethylether, Ethylengfykolethylether, Ethylenglykolpropylether, Ethylenglykolmono-nbutylether, Diethylenglykol-methylether, Diethylenglykolethylether, Propylenglykolmethyl-, - ethyl- oder -propyl-ether, Dipropylenglykolmonomethyl-, oder -ethylether, Diisopropylenglykolmonomethyl-, oder -ethylether, Methoxy-, Ethoxy- oder Butoxytriglykol, 1-Butoxyethoxy-2-propanol, 3-Methyl-3-methoxybutanol, Propylen-glykol-t-butylether Alkohole, insbesondere C₁-C₄-Alkanole, Glykole, Polyethylenglykole, vorzugsweise mit einem Molekulargewicht zwischen 100 und 100000, insbesondere zwischen 200 und 10 000, und Polyole, wie Sorbitol und Mannitol, sowie bei Raumtemperatur flüssiges Polyethylenglykol, Carbonsäureester, Polyvinylalkohole, Ethylenoxid-Propylenoxid-Blockcopolymere sowie beliebige Gemische der voranstehenden.

Zur Einstellung der Viskosität können einer flüssigen erfindungsgemäßen Zusammensetzung ein oder mehrere Verdicker bzw. Verdickungssysteme zugesetzt werden. Die Viskosität der erfindungsgemäßen Zusammensetzungen kann mit üblichen Standardmethoden (beispielsweise Brookfield-Viskosimeter RVD-VII bei 20 U/min und 20°C, Spindel 3) gemessen werden und liegt vorzugsweise im Bereich von 100 bis 5000 mPas. Bevorzugte Zusammensetzungen haben Viskositäten von 200 bis 4000 mPas, wobei Werte zwischen 400 und 2000 mPas besonders bevorzugt sind.

Geeignete Verdicker sind anorganische oder polymere organische Verbindungen. Es können auch Gemische aus mehreren Additiven eingesetzt werden.

Zu den anorganischen Verdickem zählen beispielsweise Polykieselsäuren, Tonmineralien wie Montmorillonite, Zeolithe, Kieselsäuren und Bentonite.

Die organischen Verdicker stammen aus den Gruppen der natürlichen Polymere, der abgewandelten natürlichen Polymere und der vollsynthetischen Polymere. Diese auch Quell(ungs)mittel genannten, meist hochmolekularen Stoffe saugen die Flüssigkeiten auf, quellen dabei und gehen schließlich in zähflüssige echte oder kolloide Lösungen über.

Aus der Natur stammende Polymere, die als Verdicker Verwendung finden, sind beispielsweise Agar-Agar, Carrageen, Tragant, Gummi arabicum, Alginate, Pektine, Polyosen, Guar-Mehl, Johannisbrotbaumkemmehl, Stärke, Dextrine, Gelatine und Casein.

Abgewandelte Naturstoffe stammen vor allem aus der Gruppe der modifizierten Stärken und Cellulosen, beispielhaft seien hier Carboxymethylcellulose und andere Celluloseether, Hydroxyethyl- und -propylcellulose sowie Kemmehlether genannt.

Eine große Gruppe von Verdickem, die breite Verwendung in den unterschiedlichsten Anwendungsgebieten finden, sind die vollsynthetischen Polymere wie Polyacryl- und Poiymethacryl-Verbindungen, Vinylpolymere, Polycarbonsäuren, Polyether, Polyimine, Polyamide und Polyurethane.

Die Verdicker können in einer Menge bis zu 10 Gew.-%, vorzugsweise von 0,05 bis 5 Gew.-%, und besonders bevorzugt von 0,1 bis 3 Gew.-%, bezogen auf die fertige Zusammensetzung, enthalten sein.

Darüber hinaus können auch tensidische Verdicker eingesetzt werden, z. B. Alkylpolyglycoside, wie C₈₋₁₀-Alkylpolyglucosid (APG® 220, Hersteller: Cognis Deutschland GmbH); C₁₂₋₁₄-Alkylpolyglucosid (APG® 600, Hersteller: Cognis Deutschland GmbH).

Zu den in fester Form vorliegenden Mittel zählen z. B. Pulver, Kompaktate, wie Granulate und Formkörper (Tabletten). Die einzelnen Formen können nach aus dem Stand der Technik bekannten Verfahren hergestellt werden, wie durch Sprühtrocknung, Granulation und Verpressen. Die in fester Form vorliegenden Mittel können auch in geeigneten Verpackungssystemen verpackt werden, wobei das Verpackungssystem vorzugsweise eine Feuchtigkeitsdampfdurchlässigkeitsrate von 0,1 g/m²/Tag bis weniger als 20 g/m²/Tag aufweist, wenn das Verpackungssystem bei 23°C und einer relativen Gleichgewichtsfeuchtigkeit von 85 % gelagert wird.

Die Mittel können alle für den jeweiligen Anwendungszweck üblichen Inhaltsstoffe enthalten. Als weitere Inhaltsstoffe können auch die oben als Kapselmaterialien genannten Substanzen in unverkapselter Form sowie die oben beschriebenen kationischen Verbindungen eingearbeitet werden.

Weitere mögliche Inhaltsstoffe sind Tenside und Builder (Gerüststoffe), die insbesondere in Wasch- oder Reinigungsmittel und auch in Mitteln zur Reinigung von Haut und Haaren enthalten sind. Bei den Wasch- oder Reinigungsmitteln zählen dazu üblicherweise aber auch organische Lösungsmittel Bleichmittel, Bleichaktivatoren, Enzyme, Vergrauungsinhibitoren, Schauminhibitoren, anorganische Salze, Lösungsmittel, pH-Stellmittel, Duftstoffe, Parfümträger, Fluoreszenzmittel, Farbstoffe, Hydrotope, Silikonöle, Soil-release-Verbindungen, optische Aufheller, Vergrauungsinhibitoren, Einlaufverhinderer, Knitterschutzmittel, Farbübertragungsinhibitoren, antimikrobielle Wirkstoffe. Germizide, Fungizide, Antioxidantien, Korrosionsinhibitoren, Antistatika, Bügelhilfsmittel, Phobier- und Imprägmniermittel, Quell- und Schiebefestmittel, UV-Absorber oder deren Gemische.

Die ggf. enthaltenen Tenside sind vorzugsweise ausgewählt aus nichtionischen, anionischen, amphoteren und kationischen Tensiden sowie deren beliebigen Gemischen.

Die Tenside liegen vorteillhafterweise in einer Menge von 0,1 bis 50 Gew.-%, vorzugsweise von 0,1 bis 35 Gew.-% und insbesondere von 0,1 bis 15 Gew.-%, bezogen auf die Zusammensetzung, vor.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfettoder Oleylalkohol, und durchschnittlich 2 bis 8 EO pro Mol Alkohol bevorzugt. Zu den bevorzugten ethoxylierten Alkoholen gehören beispielsweise C₁₂₋₁₄-Alkohole mit 3 EO bis 7 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 7 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO. Auch nichtionische Tenside, die EO- und PO-Gruppen zusammen im Molekül enthalten, sind erfindungsgemäß einsetzbar. Hierbei können Blockcopolymere mit EO-PO-Blockeinheiten bzw. PO-EO-Blockeinheiten eingesetzt werden, aber auch EO-PO-EO-Copolymere bzw. PO-EO-PO-Copolymere. Selbstverständlich sind auch gemischt alkoxylierte Niotenside einsetzbar, in denen EO- und PO-Einheiten nicht blockweise sondern statistisch verteilt sind. Solche Produkte sind durch gleichzeitige Einwirkung von Ethylen- und Propylenoxid auf Fettalkohole erhältlich.

Besonders bevorzugte Beispiele für nichtionische Tenside, die ein gutes Ablaufverhalten von Wasser auf harten Oberflächen bewirken, sind die Fettalkoholpolyethylenglycolether, Fettalkoholpolyethylen/polypropylenglycolether und Mischether, die ggf. endgruppeverschlossen sein können.

Beispiele für Fettalkoholpolyethylenglycolether sind solche mit der Formel (VII)

R²³O-(CH₂CH₂O)ₜH (VII)

in der R²³ für eine linearen oder verzweigten Alkyl- und/oder Alkenylrest mit 6 bis 22, vorzugsweise 12 bis 18 Kohlenstoffatomen und t für Zahlen von 1 bis 5 steht.

Die genannten Stoffe stellen bekannte Handelsprodukte dar. Typische Beispiele sind Anlagerungsprodukte von durchschnittlich 2 bzw. 4 Mol Ethylenoxid an technischen C_{12/14}-Kokosfettalkohol (Dehydol®LS-2 bzw. LS-4, Fa. Cognis Deutschland GmbH) oder Anlagerungsprodukte von durchschnittlich 4 Mol Ethylenoxid an C_{14/15}-Oxoalkohole (Dobanol®45-4, Fa. Shell). Die Produkte können eine konventionelle oder auch eingeengte Homologenverteilung aufweisen.

Unter Fettalkoholpolyethylen/polypropylengiycolethem sind nichtionische Tenside der Formel (VIII) zu verstehen, 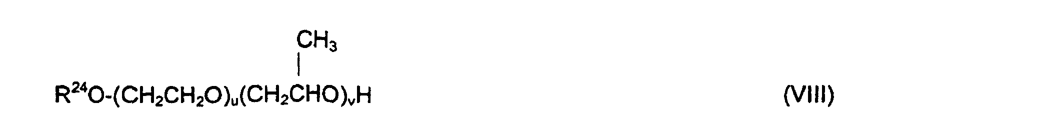 in der R²⁴ für einen linearen oder verzweigten Alkyl- und/oder Alkenylrest mit 6 bis 22, vorzugsweise 12 bis 18 Kohlenstoffatomen, u für Zahlen von 1 bis 0 und v für Zahlen von 1 bis 4 steht.

Auch diese Stoffe stellen bekannte Handelsprodukte dar. Typische Beispiele sind Anlagerungsprodukte von durchschnittlich 5 Mol Ethylenoxid und 4 Mol Propylenoxid an technischen C_{12/14}-Kokosfettalkohol (Dehydol®LS-54, Fa. Cognis Deutschland GmbH), oder 6,4 Mol Ethylenoxid und 1,2 Mol Propylenoxid an technischen C_{10/14}-Kokosfettalkohol (Dehydol® LS-980, Fa. Cognis Deutschland GmbH).

Unter Mischethem sind endgrupenverschlosssene Fettalkoholpolyglycolether mit der Formel (IX) zu verstehen 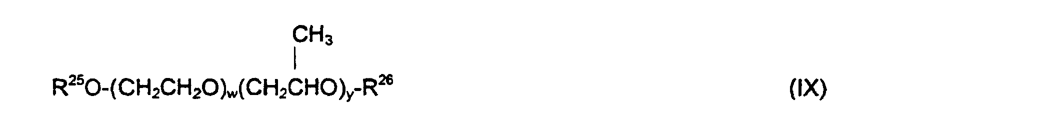 in der R²⁵ für einen linearen oder verzweigten Alkyl- und/oder Alkenylrest mit 6 bis 22, vorzugsweise 12 bis 18 Kohlenstoffatomen, w für Zahlen von 1 bis 10, y für 0 oder Zahlen von 1 bis 4 und R²⁶ für einen Alkylrest mit 1 bis 4 Kohlenstoffatomen oder einen Benzylrest steht.

Typische Beispiele sind Mischether der Formel (IX), in der R²⁵ für einen technischen C_{12/14}-Kokosalkylrest, w für 5 bzw. 10, y für 0 und R²⁶ für eine Butylgruppe steht (Dehypon®LS-54 bzw. LS-104, Fa. Cognis Deutschland GmbH). Die Verwendung von butyl- bzw. benzylgruppenverschlossenen Mischethem ist aus anwendungstechnischen Gründen besonders bevorzugt.

Unter Hydroxyalkylpolyethylenglykolethem versteht man Verbindungen mit der allgemeinen Formel (X) 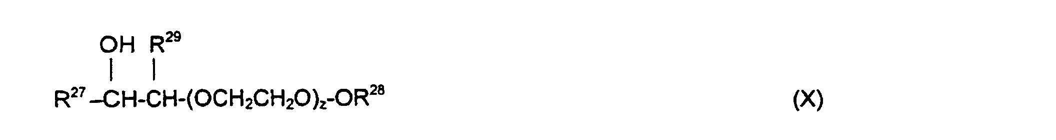 in der
- R²⁷: für Wasserstoff oder einen geradkettigen Alkylrest mit 1 bis 16 C-Atomen,
- R²⁸: für einen geradkettigen oder verzweigten Alkylrest mit 4 bis 8 C-Atomen,
- R²⁹: für Wasserstoff oder einen Alkylrest mit 1 bis 16 C-Atomen und
- z: für eine Zahl von 7 bis 30
stehen, mit der Maßgabe, daß die Gesamtzahl der in R²⁷ und R²⁹ enthaltenen C-Atome 6 bis 16 beträgt.

Außerdem können als weitere nichtionische Tenside auch Alkylglykoside der allgemeinen Formel RO(G)ₓ eingesetzt werden, in der R einen primären geradkettigen oder methylverzweigten, insbesondere in 2-Stellung methylverzweigten aliphatischen Rest mit 8 bis 22, vorzugsweise 12 bis 18 C-Atomen bedeutet und G das Symbol ist, das für eine Glykoseeinheit mit 5 oder 6 C-Atomen, vorzugsweise für Glucose, steht. Der Oligomerisierungsgrad x, der die Verteilung von Monoglykosiden und Oligoglykosiden angibt, ist eine beliebige Zahl zwischen 1 und 10; vorzugsweise liegt x bei 1,2 bis 1,4.

Eine weitere Klasse nichtionischer Tenside, die insbesondere in festen Mitteln eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein. Die Menge dieser nichtionischen Tenside beträgt vorzugsweise nicht mehr als die der ethoxylierten Fettalkohole, insbesondere nicht mehr als die Hälfte davon.

Weitere geeignete Tenside sind Polyhydroxyfettsäureamide der Formel XI, 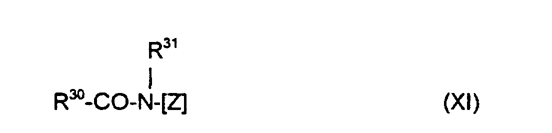 in der R³⁰CO für einen aliphatischen Acylrest mit 6 bis 22 Kohlenstoffatomen, R³¹ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zukkers mit Ammoniak, einem Alkylamin oder einem Alkanolamin und nachfolgende Acylierung mit einer Fettsäure, einem Fettsäurealkylester oder einem Fettsäurechlorid erhalten werden können.

Zur Gruppe der Polyhydroxyfettsäureamide gehören auch Verbindungen der Formel XII, 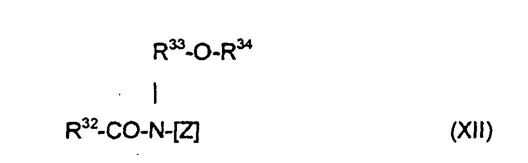 in der R³² für einen linearen oder verzweigten Alkyl- oder Alkenylrest mit 7 bis 12 Kohlenstoffatomen, R³³ für einen linearen, verzweigten oder cyclischen Alkylrest oder einen Arylrest mit 2 bis 8 Kohlenstoffatomen und R³⁴ für einen linearen, verzweigten oder cyclischen Alkylrest oder einen Arylrest oder einen Oxy-Alkylrest mit 1 bis 8 Kohlenstoffatomen steht, wobei C₁₋₄-Alkyl- oder Phenylreste bevorzugt sind und [Z] für einen linearen Polyhydroxyalkylrest steht, dessen Alkylkette mit mindestens zwei Hydroxylgruppen substituiert ist, oder alkoxylierte, vorzugsweise ethoxylierte oder propxylierte Derivate dieses Restes.

[Z] wird vorzugsweise durch reduktive Aminierung eines Zuckers erhalten, beispielsweise Glucose, Fructose, Maltose, Lactose, Galactose, Mannose oder Xylose. Die N-Alkoxyoder N-Aryloxy-substituierten Verbindungen können dann beispietweise nach der Lehre der internationalen Anmeldung WO-A-95/07331 durch Umsetzung mit Fettsäuremethylestern in Gegenwart eines Alkoxids als Katalysator in die gewünschten Polyhydroxyfettsäureamide überführt werden.

Als anionische Tenside werden beispielsweise solche vom Typ der Sulfonate und Sulfate eingesetzt. Als Tenside vom Sulfonat-Typ kommen dabei vorzugsweise C₉₋₁₃-Alkylbenzolsulfonate, Olefinsulfonate, d.h. Gemische aus Alken- und Hydroxyalkansulfonaten sowie Disulfonaten, wie man sie beispielsweise aus C₁₂₋₁₈-Monoolefinen mit endoder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, die aus C₁₂₋₁₈-Alkanen beispielsweise durch Sulfochlorierung oder Sulfoxidation mit anschließender Hydrolyse bzw. Neutralisation gewonnen werden. Ebenso sind auch die Ester von a-Sulfofettsäuren (Estersulfonate), z.B. die a-sulfonierten Methylester der hydrierten Kokos-, Palmkern- oder Talgfettsäuren geeignet.

Als Alk(en)ylsulfate werden die Alkali- und insbesondere die Natriumsalze der Schwefelsäurehalbester der C₁₂-C₁₈-Fettalkohole, beispielsweise aus Kokosfettalkohol, Talgfettalkohol, Lauryl-, Myristyl-, Cetyl- oder Stearylalkohol oder der C₁₀-C₂₀-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten. Aus waschtechnischem Interesse sind die C₁₂-C₁₆-Alkylsulfate und C₁₂-C₁₅-Alkylsulfate sowie C₁₄-C₁₅-Alkylsulfate bevorzugt. Auch 2,3-Alkylsulfate, weiche beispielsweise gemäß den US-Patentschriften 3,234,258 oder 5,075,041 hergestellt werden und als Handelsprodukte der Shell Oil Company unter dem namen DAN® erhalten werden können, sind geeignete Aniontenside.

Weitere geeignete Aniontenside sind sulfierte Fettsäureglycerinester. Unter Fettsäureglycerinestem sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Auch die Schwefelsäuremonoester der mit 1 bis 6 Mol Ethylenoxid ethoxylierten geradkettigen oder verzweigten C₇₋₂₁-Alkohole, wie 2-Methyl-verzweigte C₉₋₁₁-Alkohole mit im Durchschnitt 3,5 Mol Ethylenoxid (EO) oder C₁₂₋₁₈-Fettalkohole mit 1 bis 4 EO, sind geeignet. Sie werden in maschinellen Wasch- oder Reinigungsmitteln , insbesondere bei der Textilwäsche, aufgrund ihres hohen Schaumverhaltens nur in relativ geringen Mengen, beispielsweise in Mengen von 1 bis 5 Gew.-%. eingesetzt.

Weitere geeignete Aniontenside sind auch die Salze der Alkylsuffobemsteinsäure, die auch als Sulfosuccinate oder als Sulfobemsteinsäureester bezeichnet werden und die Monoester und/oder Diester der Sulfobemsteinsäure mit Alkoholen, vorzugsweise Fettalkoholen und insbesondere ethoxylierten Fettalkoholen darstellen. Bevorzugte Sulfosuccinate enthalten C₈₋₁₈-Fettalkoholreste oder Mischungen aus diesen. Insbesondere bevorzugte Sulfosuccinate enthalten einen Fettalkoholrest, der sich von ethoxylierten Fettalkoholen ableitet, die für sich betrachtet nichtionische Tenside darstellen (Beschreibung siehe unten). Dabei sind wiederum Sulfosuccinate, deren Fettalkohol-Reste sich von ethoxylierten Fettalkoholen mit eingeengter Homologenverteilung ableiten, besonders bevorzugt. Ebenso ist es auch möglich, Alk(en)ylbemsteinsäure mit vorzugsweise 8 bis 18 Kohlenstoffatomen in der Alk(en)ylkette oder deren Salze einzusetzen.

Als weitere anionische Tenside kommen insbesondere Seifen in Betracht, die insbesondere in pulverförmigen Mitteln und bei höheren pH-Werten eingesetzt werden. Geeignet sind gesättigte und ungesättigte Fettsäureseifen, wie die Salze der Laurinsäure, Myristinsäure, Palmitinsäure, Stearinsäure, hydrierte Erucasäure und Behensäure sowie insbesondere aus natürlichen Fettsäuren, z.B. Kokos-, Palmkem-, Olivenöl- oder Talgfettsäuren, abgeleitete Seifengemische.

Die anionischen Tenside einschließlich der Seifen können in Form ihrer Natrium-, Kaliumoder Ammoniumsalze sowie als lösliche Salze organischer Basen, wie Mono-, Di- oder Triethanolamin, vorliegen. Vorzugsweise liegen die anionischen Tenside in Form ihrer Natrium- oder Kaliumsalze, insbesondere in Form der Natriumsalze vor.

Als weitere Tenside kommen sogenannte Gemini-Tenside in Betracht. Hierunter werden im allgemeinen solche Verbindungen verstanden, die zwei hydrophile Gruppen und zwei hydrophobe Gruppen pro Molekül besitzen. Diese Gruppen sind in der Regel durch einen sogenannten "Spacer" voneinander getrennt. Dieser Spacer ist in der Regel eine Kohlenstoffkette, die lang genug sein sollte, daß die hydrophilen Gruppen einen ausreichenden Abstand haben, damit sie unabhängig voneinander agieren können. Derartige Tenside zeichnen sich im allgemeinen durch eine ungewöhnlich geringe kritische Micellkonzentration und die Fähigkeit, die Oberflächenspannung des Wassers stark zu reduzieren, aus. In Ausnahmefällen werden jedoch unter dem Ausdruck Gemini-Tenside nicht nur dimere, sondern auch trimere Tenside verstanden.

Geeignete Gemini-Tenside sind beispielsweise sulfatierte Hydroxymischether Dimeralkohol-bis- und Trimeralkohol-tris-sulfate und -ethersulfate. Endgruppenverschlossene dimere und trimere Mischether zeichnen sich insbesondere durch ihre Bi- und Multifunktionalität aus. So besitzen die genannten endgruppenverschlossenen Tenside gute Netzeigenschaften und sind dabei schaumarm, so daß sie sich insbesondere für den Einsatz in maschinellen-Wasch- oder Reinigungsverfahren eignen. Eingesetzt werden können aber auch Gemini Polyhydroxyfettsäureamide oder Poly-Polyhydroxyfettsäureamide.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Die erfindungsgemäßen Wasch- oder Reinigungsmittel können alle üblicherweise in Wasch- oder Reinigungsmitteln eingesetzten Gerüststoffe enthalten, insbesondere Zeolithe, Silikate, Carbonate, organische Cobuilder und - wo keine ökologischen Vorurteile gegen ihren Einsatz bestehen - auch die Phosphate.

Geeignete kristalline, schichtförmige Natriumsilikate besitzen die allgemeine Formel NaMSiₓO₂ₓ₊₁ H₂O, wobei M Natrium oder Wasserstoff bedeutet, x eine Zahl von 1,9 bis 4 und y eine Zahl von 0 bis 20 ist und bevorzugte Werte für x 2, 3 oder 4 sind. Bevorzugte kristalline Schichtsilikate der angegebenen Formel sind solche, in denen M für Natrium steht und x die Werte 2 oder 3 annimmt. Insbesondere sind sowohl β- als auch δ-Natriumdisilikate Na₂Si₂O₅·yH₂O bevorzugt.

Einsetzbar sind auch amorphe Natriumsilikate mit einem Modul Na₂O : SiO₂ von 1:2 bis 1:3,3, vorzugsweise von 1:2 bis 1:2,8 und insbesondere von 1:2 bis 1:2,6, weiche löseverzögert sind und Sekundärwascheigenschaften aufweisen. Die Löseverzögerung gegenüber herkömmlichen amorphen Natriumsilikaten kann dabei auf verschiedene Weise, beispielsweise durch Oberflächenbehandlung, Compoundierung, Kompaktierung/ Verdichtung oder durch Übertrocknung hervorgerufen worden sein. Im Rahmen dieser Erfindung wird unter dem Begriff "amorph" auch "röntgenamorph" verstanden. Dies heißt, daß die Silikate bei Röntgenbeugungsexperimenten keine scharfen Röntgenreflexe liefern, wie sie für kristalline Substanzen typisch sind, sondern allenfalls ein oder mehrere Maxima der gestreuten Röntgenstrahlung, die eine Breite von mehreren Gradeinheiten des Beugungswinkels aufweisen. Es kann jedoch sehr wohl sogar zu besonders guten Buildereigenschaften führen, wenn die Silikatpartikel bei Elektronenbeugungsexperimenten unscharfe oder sogar scharfe Beugungsmaxima liefern. Dies ist so zu interpretieren, daß die Produkte mikrokristalline Bereiche der Größe 10 bis einige Hundert nm aufweisen, wobei Werte bis max. 50 nm und insbesondere bis max. 20 nm bevorzugt sind. Insbesondere bevorzugt sind verdichtete/kompaktierte amorphe Silikate, compoundierte amorphe Silikate und übertrocknete röntgenamorphe Silikate.

Feinkristalliner, synthetischer und gebundenes Wasser enthaltender Zeolith ist vorzugsweise Zeolith A und/oder P. Als Zeolith P wird Zeolith MAP® (Handelsprodukt der Firma Crosfield) besonders bevorzugt. Geeignet sind jedoch auch Zeolith X sowie Mischungen aus A, X und/oder P.

Als weitere bevorzugt eingesetzte und besonders geeignete Zeolithe sind Zeolithe vom Faujasit-Typ zu nennen. Zusammen mit den Zeolithen X und Y gehört das Mineral Faujasit zu den Faujasit-Typen innerhalb der Zeolith-Strukturgruppe 4, die durch die Doppelsechsring-Untereinheit D6R gekennzeichnet ist (Vergleiche Donald W. Breck: "Zeolite Molecular Sieves", John Wiley & Sons, New York, London, Sydney, Toronto, 1974, Seite 92). Zur Zeolith-Strukturgruppe 4 zählen neben den genannten Faujasit-Typen noch die Mineralien Chabazit und Gmelinit sowie die synthetischen Zeolithe R (Chabazit-Typ), S (Gmelinit-Typ), L und ZK-5. Die beiden letztgenannten synthetischen Zeolithe haben keine mineralischen Analoga.

Zeolithe vom Faujasit-Typ sind aus β-Käfigen aufgebaut, die tetrahedral über D6R-Unterein-heiten verknüpft sind, wobei die β-Käfige ähnlich den Kohlenstoffatomen im Diamanten angeordnet sind. Das dreidimensionale Netzwerk der im erfindungsgemäßen Verfahren eingesetzten Zeolithe vom Faujasit-Typ weist Poren von 2,2 und 7,4 Å auf, die Elementarzelle enthält darüberhinaus 8 Kavitäten mit ca. 13 Å Durchmesser und läßt sich durch die Formel Na₈₆[(AlO₂)₈₆(SiO₂)₁₀₆]· 264 H₂O beschreiben. Das Netzwerk des Zeolith X enthält dabei ein Hohlraumvolumen von ungefähr 50%, bezogen auf den dehydratisierten Kristall, was den größten Leerraum aller bekannten Zeolithe darstellt (Zeolith Y: ca. 48% Hohlraumvolumen, Faujasit: ca. 47% Hohlraumvolumen). (Alle Daten aus: Donald W. Breck: "Zeolite Molecular Sieves", John Wiley & Sons, New York, London, Sydney, Toronto, 1974, Seiten 145, 176, 177).

Im Rahmen der vorliegenden Erfindung kennzeichnet der Begriff "Zeolith vom Faujasit-Typ" alle drei Zeolithe, die die Faujasit-Untergruppe der Zeolith-Strukturgruppe 4 bilden. Neben dem Zeolith X sind erfindungsgemäß also auch Zeolith Y und Faujasit sowie Mischungen dieser Verbindungen erfindungsgemäß einsetzbar, wobei der reine Zeolith X bevorzugt ist.
Auch Mischungen oder Cokristallisate von Zeolithen des Faujasit-Typs mit anderen Zeotithen, die nicht zwingend der Zeolith-Strukturgruppe 4 angehören müssen, sind erfindungs-gemäß einsetzbar, wobei die Vorteile des erfindungsgemäßen Verfahrens besonders deut-lich zu Tage treten, wenn mindestens 50 Gew.-% der Zeolithe Zeolithe vom vom Faujasit-Typ sind.

Die Aluminiumsilikate, die im erfindungsgemäßen Verfahren eingesetzt werden, sind kommerziell erhältlich, und die Methoden zu ihrer Darstellung sind in Standardmonographien be-schrieben.

Beispiele für kommerziell erhältliche Zeolithe vom X-Typ können durch die folgenden Formeln beschrieben werden:

Na₈₆[(AlO₂)₈₆(SiO₂)₁₀₆] · x H₂O,

K₈₆[(AlO₂)₈₆(SiO₂)₁₀₆] · x H₂O,

Ca₄₀Na₆[(AlO₂)₈₆(SiO₂)₁₀₆] · x H₂O,

Sr₂₁Ba₂₂[(AlO₂)₈₆(SiO₂)₁₀₆] · x H₂O,

in denen x Werte zwischen 0 und 276 annehmen kann und die Porengrößen von 8,0 bis 8,4 Å aufweisen.

Kommerziell erhältlich und im Rahmen der vorliegenden Erfindung bevorzugt einsetzbar ist beispielsweise auch ein Co-Kristallisat aus Zeolith X und Zeolith A (ca. 80 Gew.-% Zeolith X), das von der Firma CONDEA Augusta S.p.A. unter dem Markennamen VEGOBOND AX® vertrieben wird und durch die Formel

nNa₂O · (1-n)K₂O · Al₂O₃ · (2 - 2,5)SiO₂ · (3,5 - 5,5) H₂O

beschrieben werden kann. Der Zeolith kann dabei sowohl als Gerüststoff in einem granularen Compound eingesetzt, als auch zu einer Art "Abpuderung" der gesamten zu verpressenden Mischung verwendet werden, wobei üblicherweise beide Wege zur Inkorporation des Zeoliths in das Vorgemisch genutzt werden. Geeignete Zeolithe weisen eine mittlere Teilchengröße von weniger als 10 µm (Volumenverteilung; Meßmethode: Coulter Counter) auf und enthalten vorzugsweise 18 bis 22 Gew.-%, insbesondere 20 bis 22 Gew.-% an gebundenem Wasser.

Selbstverständlich ist auch ein Einsatz der allgemein bekannten Phosphate als Buildersubstanzen möglich, sofern ein derartiger Einsatz nicht aus ökologischen Gründen vermieden werden sollte. Unter der Vielzahl der kommerziell erhältlichen Phosphate haben die Alkalimetallphosphate unter besonderer Bevorzugung von Pentanatrium- bzw. Pentakaliumtriphosphat (Natrium- bzw. Kaliumtripolyphosphat) in der Wasch- oder Reinigungsmittel-Industrie die größte Bedeutung. Sie werden heute vor allem in maschinellen Geschirrspülmitteln eingesetzt.

Alkalimetallphosphate ist dabei die summarische Bezeichnung für die Alkalimetall- (insbesondere Natrium- und Kalium-) -Salze der verschiedenen Phosphorsäuren, bei denen man Metaphosphorsäuren (HPO₃)ₙ und Orthophosphorsäure H₃PO₄ neben höhermolekularen Vertretern unterscheiden kann. Die Phosphate vereinen dabei mehrere Vorteile in sich: Sie wirken als Alkaliträger, verhindern Kalkbeläge auf Maschinenteilen bzw. Kalkinkrustationen in Geweben und tragen überdies zur Reinigungsleistung bei.

Als organische Cobuilder können in den erfindungsgemäßen Wasch- oder Reinigungsmittelformkörpern insbesondere Polycarboxylate / Polycarbonsäuren, polymere Polycarboxylate, Asparaginsäure, Polyacetale, Dextrine, weitere organische Cobuilder (siehe unten) sowie Phosphonate eingesetzt werden. Diese Stoffklassen werden nachfolgend beschrieben.

Brauchbare organische Gerüstsubstanzen sind beispielsweise die in Form ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bemsteinsäure, Glutarsäure, Äpfelsäure, Weinsäure, Maleinsäure, Fumarsäure, Zuckersäuren. Aminocarbonsäuren, Nitrilotriessigsäure (NTA), sofern ein derartiger Einsatz aus ökologischen Gründen nicht zu beanstanden ist, sowie Mischungen aus diesen. Bevorzugte Salze sind die Salze der Polycarbonsäuren wie Citronensäure, Adipinsäure, Bernsteinsäure. Glutarsäure, Weinsäure, Zuckersäuren und Mischungen aus diesen.

Auch die Säuren an sich können eingesetzt werden. Die Säuren besitzen neben ihrer Builderwirkung typischerweise auch die Eigenschaft einer Säuerungskomponente und dienen somit auch zur Einstellung eines niedrigeren und milderen pH-Wertes von Waschoder Reinigungsmitteln, Insbesondere sind hierbei Citronensäure, Bemsteinsäure, Glutarsäure, Adipinsäure, Gluconsäure und beliebige Mischungen aus diesen zu nennen.

Als Builder sind weiter polymere Polycarboxylate geeignet, dies sind beispielsweise die Alkalimetallsalze der Polyacrylsäure oder der Polymethacrylsäure, beispielsweise solche mit einer relativen Molekülmasse von 500 bis 70000 g/mol.

Bei den für polymere Polycarboxylate angegebenen Molmassen handelt es sich im Sinne dieser Schrift um gewichtsmittlere Molmassen M_{w} der jeweiligen Säureform, die grundsätzlich mittels Gelpermeationschromatographie (GPC) bestimmt wurden, wobei ein UV-Detektor eingesetzt wurde. Die Messung erfolgte dabei gegen einen externen Polyacrylsäure-Standard, der aufgrund seiner strukturellen Verwandtschaft mit den untersuchten Polymeren realistische Molgewichtswerte liefert. Diese Angaben weichen deutlich von den Molgewichtsangaben ab, bei denen Polystyrolsulfonsäuren als Standard eingesetzt werden. Die gegen Polystyrolsulfonsäuren gemessenen Molmassen sind in der Regel deutlich höher als die in dieser Schrift angegebenen Molmassen.

Geeignete Polymere sind insbesondere Polyacrylate, die bevorzugt eine Molekülmasse von 2000 bis 20000 g/mo! aufweisen. Aufgrund ihrer überlegenen Löslichkeit können aus dieser Gruppe wiederum die kurzkettigen Polyacrylate, die Molmassen von 2000 bis 10000 g/mol, und besonders bevorzugt von 3000 bis 5000 g/mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molekülmasse, bezogen auf freie Säuren, beträgt im allgemeinen 2000 bis 70000 g/mol, vorzugsweise 20000 bis 50000 g/mol und insbesondere 30000 bis 40000 g/mol.

Die (co-)polymeren Polycarboxylate können entweder als Pulver oder als wäßrige Lösung eingesetzt werden. Der Gehalt der Mittel an (co-)polymeren Polycarboxylaten beträgt vorzugsweise 0,5 bis 20 Gew.-%, insbesondere 3 bis 10 Gew.-%.

Zur Verbesserung der Wasserlöslichkeit können die Polymere auch Allylsulfansäuren, wie beispielsweise Allyloxybenzolsulfonsäure und Methallylsulfonsäure, als Monomer enthalten.

Insbesondere bevorzugt sind auch biologisch abbaubare Polymere aus mehr als zwei verschiedenen Monomereinheiten, beispielsweise solche, die als Monomere Salze der Acrylsäure und der Maleinsäure sowie Vinylalkohol bzw. Vinylalkohol-Derivate oder die als Monomere Salze der Acrylsäure und der 2-Alkylallylsuifonsäure sowie Zucker-Derivate enthalten.

Weitere bevorzugte Copolymere sind solche, die als Monomere vorzugsweise Acrolein und Acrylsäure/Acrylsäuresalze bzw. Acrolein und Vinylacetat aufweisen.

Ebenso sind als weitere bevorzugte Buildersubstanzen polymere Aminodicarbonsäuren, deren Salze oder deren Vorläufersubstanzen zu nennen. Besonders bevorzugt sind Polyasparaginsäuren bzw. deren Salze und Derivate.

Weitere geeignete Buildersubstanzen sind Polyacetale, welche durch Umsetzung von Dialdehyden mit Polyolcarbonsäuren, welche 5 bis 7 C-Atome und mindestens 3 Hydroxylgruppen aufweisen, erhalten werden können. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Weitere geeignete organische Buildersubstanzen sind Dextrine, beispielsweise Oligomere bzw. Polymere von Kohlenhydraten, die durch partielle Hydrolyse von Stärken erhalten werden können. Die Hydrolyse kann nach üblichen, beispielsweise säure- oder enzymkatalysierten Verfahren durchgeführt werden. Vorzugsweise handelt es sich um Hydrolyseprodukte mit mittleren Molmassen im Bereich von 400 bis 500000 g/mol. Dabei ist ein Polysaccharid mit einem Dextrose-Äquivalent (DE) im Bereich von 0,5 bis 40, insbesondere von 2 bis 30 bevorzugt, wobei DE ein gebräuchliches Maß für die reduzierende Wirkung eines Polysaccharids im Vergleich zu Dextrose, welche ein DE von 100 besitzt, ist. Brauchbar sind sowohl Maltodextrine mit einem DE zwischen 3 und 20 und Trockenglucosesirupe mit einem DE zwischen 20 und 37 als auch sogenannte Gelbdextrine und Weißdextrine mit höheren Molmassen im Bereich von 2000 bis 30000 g/mol.

Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren. Ein an C₆ des Saccharidrings oxidiertes Produkt kann besonders vorteilhaft sein.

Auch Oxydisuccinate und andere Derivate von Disuccinaten, vorzugsweise Ethylendiamindisuccinat, sind weitere geeignete Cobuilder. Dabei wird Ethylendiamin-N,N'disuccinat (EDDS) bevorzugt in Form seiner Natrium- oder Magnesiumsalze verwendet. Weiterhin bevorzugt sind in diesem Zusammenhang auch Glycerindisuccinate und Glycerintrisuccinate. Geeignete Einsatzmengen liegen in zeolithhaltigen und/oder silicathaltigen Formulierungen bei 3 bis 15 Gew.-%.

Weitere brauchbare organische Cobuilder sind beispielsweise acetylierte Hydroxycarbonsäuren bzw. deren Salze, welche gegebenenfalls auch in Lactonform vorliegen können und welche mindestens 4 Kohlenstoffatome und mindestens eine Hydroxygruppe sowie maximal zwei Säuregruppen enthalten.

Eine weitere Substanzklasse mit Cobuildereigenschaften stellen die Phosphonate dar. Dabei handelt es sich insbesondere um Hydroxyalkan- bzw. Aminoalkanphosphonate. Unter den Hydroxyalkanphosphonaten ist das 1-Hydroxyethan-1,1-diphosphonat (HEDP) von besonderer Bedeutung als Cobuilder. Es wird vorzugsweise als Natriumsalz eingesetzt, wobei das Dinatriumsalz neutral und das Tetranatriumsalz alkalisch (pH 9) reagiert. Als Aminoalkanphosphortate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z. B. als Hexanatriumsalz der EDTMP bzw. als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoaikanphosphonate, insbesondere DTPMP, einzusetzen, oder Mischungen aus den genannten Phosphonaten zu verwenden.

Darüber hinaus können alle Verbindungen, die in der Lage sind, Komplexe mit Erdalkaliionen auszubilden, als Cobuilder eingesetzt werden.

Weiterhin können die erfindungsgemäßen Mittel alle üblicherweise in solchen Mitteln enthaltenen Substanzen aufweisen. In Wasch- oder Reinigungsmitteln können z.B. Enzyme, Bleichmittel, Bleichaktivatoren, Vergrauungsinhibitoren, Schauminhibitoren, anorganische Salze, Duftstoffe, Parfümträger, Fluoreszenzmittel, Farbstoffe, Hydrotope, Silikonöle, Soilreiease-Verbindungen, optische Aufheller, Vergrauungsinhibitoren, Einlaufverhinderer, Knitterschutzmittel, Farbübertragungsinhibitoren, antimikrobielle Wirkstoffe, Germizide, Fungizide, Antioxidantien, Korrosionsinhibitoren, Antistatika, Bügelhilfsmittel, Phobier- und Imprägniermittel. Quell- und Schiebefestmittel, Fettstoffe, Lecithin, Konservierungsmittel, Perlglanzmittel. UV-Absorber oder deren Gemische enthalten sein. Weiterhin können die Mittel Elektrolyte enthalten, vorzugsweise Natrium-, Magnesium- oder Calcium- Carbonate, -Sulfate oder -Chloride, sowie pH-Stellmittel, wie organische oder anorganische Säuren.

Als in den Mitteln verwendbare Enzyme kommen solche aus der Klasse der Oxidasen, Proteasen, Lipasen, Cutinasen, Amylasen, Pullulanasen, Cellulasen, Hemicellulasen, Xylanasen und Peroxidasen sowie deren Gemische in Frage, beispielsweise Proteasen wie BLAP®, Optimase®, Opticlean®, Maxacal®, Maxapem®, Alcalase®, Esperase® und/oder Savinase®, Amylasen wie Termamyl®, Amylase-LT®, Maxamyl®, Duramyl® und/oder Purafect® OxAm, Lipasen wie Lipolase®, Lipomax®, Lumafast® und/oder Lipozym®, Cellulasen wie Celiuzyme® und oder Carezyme®. Besonders geeignet sind aus Pilzen oder Bakterien, wie Bacillus subtilis, Bacillus licheniformis, Streptomyces griseus, Humicola lanuginosa, Humicola insolens, Pseudomonas pseudoalcaligenes oder Pseudomonas cepacia gewonnene enzymatische Wirkstoffe. Die gegebenenfalls verwendeten Enzyme können, wie zum Beispiel in der europäischen Patentschrift EP 0 564476 oder in der internationalen Patentanmeldungen WO 94/23005 beschrieben, an Trägerstoffen adsorbiert und/oder in Hüllsubstanzen eingebettet sein, um sie gegen vorzeitige Inaktivierung zu schützen. Sie sind in den erfindungsgemäßen Tensidmischungen vorzugsweise in Mengen bis zu 10 Gew.-%, insbesondere von 0,2 Gew.-% bis 2 Gew.-%, enthalten, wobei besonders bevorzugt gegen oxidativen Abbau stabilisierte Enzyme.

Unter den als Bleichmittel dienenden, in Wasser H₂O₂ liefernden Verbindungen haben das Natriumpercarbonat, das Natriumperborattetrahydrat und das Natriumperboratmonohydrat besondere Bedeutung. Weitere brauchbare Bleichmittel sind beispielsweise Peroxopyrophosphate, Citratperhydrate sowie H₂O₂ liefernde persaure Salze oder Persäuren, wie Persulfate wie die unter der Handelsbezeichnung CAROAT® erhältlichen Salze beziehungsweise Perschwefelsäure. Brauchbar ist auch das Hamstoffperoxohydrat Percarbamid, das durch die Formel H₂N-CO-NH₂·H₂O₂ beschrieben werden kann. Insbesondere beim Einsatz der Mittel für das Reinigen harter Oberflächen, zum Beispiel beim maschinellen Geschirrspülen, können sie gewünschtenfalls auch Bleichmittel aus der Gruppe der organischen Bleichmittel enthalten, obwohl deren Einsatz prinzipiell auch bei Mitteln für die Textilwäsche möglich ist. Typische organische Bleichmittel sind die Diacylperoxide, wie zum Beispiel Dibenzoylperoxid. Weitere typische organische Bleichmittel sind die Peroxysäuren, wobei als Beispiele besonders die Alkylperoxysäuren und die Arylperoxysäuren genannt werden. Bevorzugte Vertreter sind die Peroxybenzoesäure und ihre ringsubstituierten Derivate, wie Alkylperoxybenzoesäuren, aber auch Peroxy-α-Naphtoesäure und Magnesium-monoperphthalat, die aliphatischen oder substituiert aliphatischen Peroxysäuren, wie Peroxylaurinsäure, Peroxystearinsäure, ε-Phthatimidoperoxycapronsäure (Phthatimidoperoxyhexansäure, PAP), o-Carboxybenzamidoperoxycapronsäure, N-Nonenylamidoperadipinsäure und N-Nonenylamidopersuccinate, und aliphatische und araliphatische Peroxydicarbonsäuren, wie 1,12-Diperoxycarbonsäure, 1,9-Diperoxyazelainsäure, Diperoxysebacinsäure, Diperoxybrassylsäure, die Diperoxyphthalsäuren, 2-Decyldiperoxybutan-1,4-disäure, N,N-Terephthaioyl-di(6-aminopercapronsäure) können eingesetzt werden. Vorzugsweise werden die Bleichmittel, insbesondere organische Persäuren, Alkaliperborate und/oder Alkalipercarbonate, in Mengen von 0,1 bis 40 Gew.-%. vorteilhafterweise von 3 bis 30 Gew.-% und insbesondere von 5 bis 25 Gew.-% eingesetzt.

Um beim Waschen und Reinigen bei Temperaturen von 60 °C und darunter, und insbesondere bei der Wäschevorbehandlung eine verbesserte Bleichwirkung zu erreichen, können Bleichaktivatoren in die Wasch- oder Reinigungsmittelformkörper eingearbeitet werden. Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glycolurile, insbesondere 1,3,4,6-Tetraacetyiglycoluril (TAGU), N-Acylimide, insbesondere N-Nonanoylsuccinimid (NOSI), acylierte Phenolsulfonate, insbesondere *n*-Nonanoyl- oder Isononanoyloxybenzotsulfonat (*n*- bzw. iso-NOBS), acylierte Hydroxycarbonsäuren, wie Triethyl-O-acetylcitrat (TEOC), Carbonsäureanhydride, insbesondere Phthalsäureanhydrid, Isatosäureanhydrid und/oder Bernsteinsäureanhydrid, Carbonsäureamide, wie N-Methyldiacetamid, Glycolid, acylierte mehrwertige Alkohole, insbesondere Triacetin, Ethylenglycoldiacetat, Isopropenytacetat, 2,5-Diacetoxy-2,5-dihydrofuran und Enolester sowie acetyliertes Sorbitol und Mannitol beziehungsweise Gemische, die diese Substanzen enthalten, acylierte Zuckerderivate, insbesondere Pentaacetylglucose (PAG), Pentaacetylfructose, Tetraacetylxylose und Octaacetyllactose sowie acetyliertes, gegebenenfalls N-alkyliertes Glucamin bzw. Gluconolacton, Triazol bzw. Triazotderivate und/oder teilchenförmige Caprolactame und/oder Caprolactamderivate, bevorzugt N-acytierte Lactame, beispielsweise N-Benzoylcaprolactam und N-Acetylcaprolactam. Hydrophil substituierte Acylacetale Acyllactame können ebenfalls bevorzugt eingesetzt werden. Auch Kombinationen konventioneller Bleichaktivatoren können eingesetzt werden. Ebenso können Nitrilderivate wie Cyanopyridine, Nitrilquats, z. B. N-Alkyammoniumacetonitrile, und/oder Cyanamidderivate eingesetzt werden. Bevorzugte Bleichaktivatoren sind Natrium-4-(octanoyloxy)-benzolsulfonat, *n*-Nonanoyl- oder Isononanoyloxybenzolsulfonat (*n*- bzw. iso-NOBS), Undecenoytoxybenzotsulfonat (UDOBS), Natriumdodecanoyloxybenzolsulfonat (DOBS), Decanoyloxybenzoesäure (DOBA, OBC 10) und/oder Dodecanoyloxybenzolsulfonat (OBS 12), sowie N-Methyimorpholinumacetonitril (MMA). Derartige Bleichaktivatoren sind im üblichen Mengenbereich von 0,01 bis 20 Gew.-%, vorzugsweise in Mengen von 0,1 bis 15 Gew.-%, insbesondere 1 Gew.-% bis 10 Gew.-%, bezogen auf die gesamte Zusammensetzung, enthalten.

Als Bleichmittel können im Prinzip auch Chlor oder Brom freisetzende Substanzen eingesetzt werden. Diese sind jedoch für den Einsatz in Waschmitteln von geringer Bedeutung. Unter den geeigneten Chlor oder Brom freisetzenden Materialien kommen beispielsweise heterocyclische N-Brom- und N-Chloramide, beispielsweise Trichlorisocyanursäure, Tribromisocyanursäure, Dibromisocyanursäure und/oder Dichlorisocyanursäure (DICA) und/oder deren Salze mit Kationen wie Kalium und Natrium in Betracht. Hydantoinverbindungen, wie 1,3-Dichlor-5,5-dimethylhydanthoin sind ebenfalls geeignet.

Zusätzlich zu den konventionellen Bleichaktivatoren oder an deren Stelle können auch sogenannte Bleichkatalysatoren enthalten sein. Bei diesen Stoffen handelt es sich um bleichverstärkende Übergangsmetallsalze bzw. Übergangsmetallkomplexe wie beispielsweise Mn-, Fe-, Co-, Ru - oder Mo-Salenkomplexe oder -carbonylkomplexe. Auch Mn-, Fe-, Co-, Ru-, Mo-, Ti-, V- und Cu-Komplexe mit N-haltigen Tripod-Liganden sowie Co-, Fe-, Cu- und Ru-Amminkomplexe sind als Bleichkatalysatoren geeignet.

Weitere denkbare Zusätze sind Schauminhibitoren wie zum Beispiel schauminhibierendes Paraffinöl oder schauminhibierendes Silikonöl, beispielsweise Dimethylpolysiloxan. Auch der Einsatz von Mischungen dieser Wirkstoffe ist möglich. Als bei Raumtemperatur feste Zusatzstoffe kommen, insbesondere bei den genannten schauminhibierenden Wirkstoffen, Paraffinwachse, Kieselsäuren, die auch in bekannter Weise hydrophobiert sein können, und von C₂₋₇-Diaminen und C₁₂₋₂₂-Carbonsäuren abgeleitete Bisamide in Frage.

Für den Einsatz in Frage kommende schauminhibierende Paraffinöle, die in Abmischung mit Paraffinwachsen vorliegen können, stellen im allgemeinen komplexe Stoffgemische ohne scharfen Schmelzpunkt dar. Zur Charakterisierung bestimmt man üblicherweise den Schmelzbereich durch Differential-Thermo-Analyse (DTA), wie in "The Analyst"87 (1962), 420, beschrieben, und/oder den Erstarrungspunkt. Darunter versteht man die Temperatur, bei der das Paraffin durch langsames Abkühlen aus dem flüssigen in den festen Zustand übergeht. Paraffine mit weniger als 17 C-Atomen sind erfindungsgemäß nicht brauchbar, ihr Anteil im Paraffinölgemisch sollte daher so gering wie möglich sein und liegt vorzugsweise unterhalb der mit üblichen analytischen Methoden, zum Beispiel Gaschromatographie, signifikant meßbaren Grenze. Vorzugsweise werden Paraffine verwendet, die im Bereich von 20°C bis 70°C erstarren. Dabei ist zu beachten, daß auch bei Raumtemperatur fest erscheinende Paraffinwachsgemische unterschiedliche Anteile an flüssigen Paraffinölen enthalten können. Bei den erfindungsgemäß brauchbaren Paraffinwachsen liegt der Flüssiganteil bei 40°C möglichst hoch, ohne bei dieser Temperatur schon 100 % zu betragen. Bevorzugte Paraffinwachsgemische weisen bei 40°C einen Flüssiganteil von mindestens 50 Gew.-%, insbesondere von 55 Gew.-% bis 80 Gew.-%, und bei 60°C einen Flüssiganteil von mindestens 90 Gew.-% auf. Dies hat zur Folge, daß die Paraffine bei Temperaturen bis hinunter zu mindestens 70°C, vorzugsweise bis hinunter zu mindestens 60°C fließfähig und pumpbar sind. Außerdem ist darauf zu achten, daß die Paraffine möglichst keine flüchtigen Anteile enthalten. Bevorzugte Paraffinwachse enthalten weniger als 1 Gew.-%, insbesondere weniger als 0,5 Gew.-% bei 110°C und Normaldruck verdampfbare Anteile. Erfindungsgemäß brauchbare Paraffine können beispielsweise unter den Handelsbezeichnungen Lunaflex® der Firma Futter sowie Deawax® der DEA Mineralöl AG bezogen werden.

Die Paraffinöle können bei Raumtemperatur feste Bisamide, die sich von gesättigten Fettsäuren mit 12 bis 22, vorzugsweise 14 bis 18 C-Atomen sowie von Alkylendiaminen mit 2 bis 7 C-Atomen ableiten, enthalten. Geeignete Fettsäuren sind Laurin-, Myristin-, Stearin-, Arachin- und Behensäure sowie deren Gemische, wie sie aus natürlichen Fetten beziehungsweise gehärteten Ölen, wie Talg oder hydriertem Palmöl, erhältlich sind. Geeignete Diamine sind beispielsweise Ethylendiamin 1,3-Propylendiamin, Tetramethylendiamin, Pentamethylendiamin, Hexamethylendiamin, p-Phenylendiamin und Toluylendiamin. Bevorzugte Diamine sind Ethylendiamin und Hexamethylendiamin. Besonders bevorzugte Bisamide sind Bis-myristoyl-ethylendiamin, Bispalmitoyl-ethylendiamin, Bis-stearoylethylendiamin und deren Gemische sowie die entsprechenden Derivate des Hexamethylendiamins.

Weiterhin können Farbstoffe, insbesondere wasserlösliche oder wasserdispergierbare Farbstoffe enthalten sein. Bevorzugt sind hier Farbstoffe, wie sie zur Verbesserung der optischen Produktanmutung in Wasch- oder Reinigungsmittel üblicherweise eingesetzt werden. Die Auswahl derartiger Farbstoffe bereitet dem Fachmann keine Schwierigkeiten, insbesondere da derartige übliche Farbstoffe eine hohe Lagerstabilität und Unempfindlichkeit gegenüber den übrigen Inhaltsstoffen der waschaktiven Zubereitungen und gegen Licht sowie keine ausgeprägte Substantivität gegenüber Textitfasem haben, um diese nicht anzufärben.

Beispiele für optische Aufheller sind Derivate von Diaminostilbendisulfonsäure beziehungsweise deren Alkalimetallsalze. Geeignet sind z. B. Salze der 4, 4'-Bis(2-anilino-4-morpholinol,3,5-triazinyl-6-amino)stilben-2,2'-disulfonsäure oder gleichartig aufgebaute Verbindungen, die anstelle der Morpholino-Gruppe eine Diethanoiamino-Gruppe, eine Methyiamino-Gruppe, eine Anilino-Gruppe oder eine 2-Methoxyethylamino-Gruppe tragen. Weiterhin können Aufheller vom Typ der substituierten Diphenyistyryle in den Teil-Portionen (waschaktiven Zubereitungen) der erfindungsgemäßen Wasch- oder Reinigungsmittel-Portionen enthalten sein, z. B. die Alkalisalze des 4,4'-Bis(2-sulfostyryl)-diphenyls, 4,4'-Bis(4-chlor-3-sulfostyryl)diphenyls oder 4-(4-Chlorstyryl)4'-(2-sulfostyryl)-diphenyls. Auch Gemische der vorgenannten Aufheller können verwendet werden.

Die Mittel können UV-Absorber enthalten, die auf die behandelten Textilien aufziehen und die Lichtbeständigkeit der Fasern und/oder die Lichtbeständigkeit des sonstiger Rezepturbestandteile verbessern. Unter UV-Absorber sind organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. Verbindungen, die diese gewünschten Eigenschaften aufweisen, sind beispielsweise die durch strahlungslose Desaktivierung wirksamen Verbindungen und Derivate des Benzophenons mit Substituenten in 2- und/oder 4-Stellung. Weiterhin sind auch substituierte Benzotriazole, in 3-Stellung Phenylsubstituierte Acrylate (Zimisäurederivate), gegebenenfalls mit Cyanogruppen in 2-Stellung, Salicylate, organische Ni-Komplexe sowie Naturstoffe wie Umbelliferon und die körpereigene Urocansäure geeignet. Besondere Bedeutung haben Biphenyl- und vor allem Stilbenderivate wie sie beispielsweise in der EP 0728749 A beschrieben werden und kommerziell als Tinosorb® FD oder Tinosorb® FR ex Ciba erhältlich sind. Als UV-B-Absorber sind zu nennen 3-Benzylidencampher bzw. 3-Benzylidennorcampher und dessen Derivate, z.B. 3-(4-Methylbenzyliden)campher, wie in der EP 0693471 B1 beschrieben; 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester, Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3,3-phenylzimtsäure-2-ethylhexylester (Octocrylene); Ester der Salicylsäure, vorzugsweise Salicylsäure-2-ethylhexylester, Salicylsäure-4-isopropylbenzylester, Salicylsäurehomomenthylester; Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon; Ester der Benzalmalonsäure, vorzugsweise 4-Methoxybenzmalonsäuredi-2-ethylhexylester; Triazinderivate, wie z.B. 2,4,6-Trianilino-(pcarbo-2'-ethyt-1'-hexyloxy)-1,3,5-triazin und Octyl Triazon, wie in der EP 0818450 A1 beschrieben oder Dioctyl Butamido Triazone (Uvasorb® HEB); Propan-1,3-dione, wie z.B. 1-(4-tert.Butylphenyl)-3-(4'methoxyphenyl)propan-1,3-dion; Ketotricyclo(5.2.1.0)decan-Derivate, wie in der EP 0694521 B1 beschrieben. Weiterhin geeignet sind 2-Phenylbenzimidazol-5-sulfonsäure und deren Alkali-, Erdalkali-, Ammonium-, Alkylammonium-, Alkanolammonium- und Glucammoniumsalze; Sulfonsäurederivate von Benzophenonen, vorzugsweise 2-Hydroxy-4-methoxybenzophenon-5-sulfonsäure und ihre Salze; Sulfonsäurederivate des 3-Benzylidencamphers, wie z.B. 4-(2-Oxo-3-bomylidenmethyl)benzol-sulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beschrieben in der DE 19712033 A1 (BASF). Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse, vorzugsweise nanoisierte Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente bereits für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. Vorzugsweise wird mikronisiertes Zinkoxid verwendet. Weitere geeignete UV-Lichtschutzfilter sind der Übersicht von P.Finkel in SÖFW-Journal 122, 543 (1996) zu entnehmen.

Die UV-Absorber werden üblicherweise in Mengen von 0,01 Gew.-% bis 5 Gew.-%, vorzugsweise von 0,03 Gew.-% bis 1 Gew.-%, eingesetzt.

Eine weitere Klasse von üblichen Wasch- und Reinigungsmittelinhaltsstoffen sind Polymere. Unter diesen Polymeren kommen zum einen Polymere in Frage, die beim Waschen oder Reinigen beziehungsweise Spülen Cobuilder-Eigenschaften zeigen. Diese wurden oben bereits eingehend beschrieben.

Eine weitere Gruppe von Polymeren sind Vergrauungsinhibitoren. Diese haben die Aufgabe, den von der harten Oberfläche und insbesondere von der Textilfaser abgelösten Schmutz in der Flotte suspendiert zu halten und so den Co-Builder zu unterstützen. Hierzu sind wasserlösliche Kolloide meist organischer Natur geeignet, beispielsweise Leim, Gelatine, Salze von Ethercarbonsäuren oder Ethersulfonsäuren der Stärke oder der Cellulose oder Salze von sauren Schwefelsäureestem der Cellulose oder der Stärke. Auch wasserlösliche, saure Gruppen enthaltende Polyamide sind für diesen Zweck geeignet. Weiterhin lassen sich andere als die obengenannten Stärkeprodukte verwenden, zum Beispiel Aldehydstärken. Bevorzugt werden Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethyicellulose und deren Gemische, beispielsweise in Mengen von 0,1 bis 5 Gew.-%, bezogen auf die hoch sodahaltigen Partikel, eingesetzt.

Als weitere Zusätze können die erfindungsgemäßen Mittel auch Antiredepositionsmittel, sogenannte Soil Repellents, enthalten. Dies sind Polymere, die auf Fasern oder harte Oberflächen aufziehen und dort einer Wiederanschmutzung entgegenwirken. Dieser Effekt wird besonders deutlich, wenn ein Textil verschmutzt wird, das bereits vorher mehrfach mit einem erfindungsgemäßen Waschmittel, das diese öl- und fettlösende Komponente enthält, gewaschen wurde. Zu den bevorzugten öl- und fettlösenden Komponenten zählen beispielsweise nichtionische Celluloseether wie Methylcellulose und Methylhydroxypropylcellulose mit einem Anteil an Methoxy-Gruppen von 15 bis 30 Gew.-% und an Hydroxypropoxyl-Gruppen von 1 bis 15 Gew.-%, jeweils bezogen auf den nichtionischen Celluloseether, sowie die aus dem Stand der Technik bekannten Polymere der Phthalsäure und/oder der Terephthalsäure beziehungsweise von deren Derivaten, insbesondere Polymere aus Ethylenterephthalaten und/oder Polyethylenglykolterephthalaten oder anionisch und/oder nichtionisch modifizierten Derivaten von diesen. Besonders bevorzugt von diesen sind die sulfonierten Derivate der Phthalsäure- und der Terephthalsäure-Polymere.

Weitere in Frage kommende Polymere sind Farbübertragungsinhibitoren. Dazu gehören insbesondere Polyvinylpyrrolidone, Polyvinylimidazole, polymere N-Oxide wie Poly(vinylpyridin-N-oxid) und Copolymere von Vinylpyrrolidon mit Vinylimidazol.

Zur Bekämpfung von Mikroorganismen können die Wasch- oder Reinigungsmittel antimikrobielle Wirkstoffe enthalten. Hierbei unterscheidet man je nach antimikrobiellem Spektrum und Wirkungsmechanismus zwischen Bakteriostatika und Bakteriziden, Fungistatika und Fungiziden usw. Wichtige Stoffe aus diesen Gruppen sind beispielsweise Benzalkoniumchloride, Alkylarylsulfonate, Halogenphenole und Phenolmercuriacetat. Die Begriffe antimikrobielle Wirkung und antimikrobieller Wirkstoff haben im Rahmen der erfindungsgemäßen Lehre die fachübliche Bedeutung, die beispielsweise von *K*. *H*. *Wallhäußer* in "Praxis der Sterilisation, Desinfektion - Konservierung: Keimidentifizierung - Betriebshygiene" (5. Auf). - Stuttgart; New York : Thieme, 1995) wiedergegeben wird, wobei alle dort beschriebenen Substanzen mit antimikrobieller Wirkung eingesetzt werden können. Geeignete antimikrobiette Wirkstoffe sind vorzugsweise ausgewählt aus den Gruppen der Alkohole, Amine, Aldehyde, antimikrobiellen Säuren bzw. deren Salze, Carbonsäureester, Säureamide, Phenole, Phenolderivate, Diphenyle, Diphenylalkane, Hamstoffderivate, Sauerstoff-, Stickstoff-acetale sowie -formale, Benzamidine, Isothiazoline, Phthalimidderivate, Pyridinderivate, antimikrobiellen oberflächenaktiven Verbindungen, Guanidine, antimikrobiellen amphoteren Verbindungen, Chinoline, 1,2-Dibrom-2,4-dicyanobutan, lodo-2-propyl-butyl-carbamat, lod, lodophore, Peroxoverbindungen, Halogenverbindungen sowie beliebigen Gemischen der voranstehenden.

Der antimikrobielle Wirkstoff kann dabei ausgewählt sein aus Ethanol, n-Propanol, i-Propanal, 1,3-Butandiol, Phenoxyethanol, 1,2-Propylenglykol. Glycerin, Undecylensäure, Benzossäure, Salicylsäure, Dihydracetsäure, o-Phenylphenol, N-Methylmorpholinacetonitril (MMA), 2-Benzyl-4-chlorphenol, 2,2'-Methylen-bis-(6-brom-4-chlorphenol), 4,4'-Dichlor-2'-hydroxydiphenylether (Dichtosan), 2,4,4'-Trichlor-2'-hydroxydiphenylether (Trichlosan), Chlorhexidin, N-(4-Chlorphenyl)-N-(3,4-dichlorphenyl)-hamstoff, N,N'-(1,10-decan-diyldi-1-pyridinyl-4-yliden)-bis-(1-octanamin)-dihydrochlorid, N,N'-Bis-(4-chlorphenyl)-3,12-diimino-2,4,11,13-tetraaza-tetradecandiimidamid, Glucoprotaminen, antimikrobiellen oberflächenaktiven quaternären Verbindungen, Guanidinen einschl. den Bi- und Polyguanidinen, wie beispielsweise 1,6-Bis-(2-ethylhexyl-biguanido-hexan)-dihydrochlorid, 1,6-Di-(N₁,N₁'-phenyldiguanido-N₅,N₅')-hexan-tetrahydochlorid, 1,6-Di-(N₁,N₁'-phenyl-N₁,N₁-methyldiguanido-N₅,N₅')-hexan-dihydrochlorid, 1,6-Di-(N₁,N₁'-o-chlorophenyldiguanido- N₅,N₅')-hexan-dihydrochlorid, 1,6-Di-(N₁,N₁'-2,6-dichlorophenyldiguanido-N₅,N₅')hexan-dihydrochlorid, 1,6-Di-[N₁,N₁'-beta-(p-methoxyphenyl) diguanido-N₅,N₅']-hexane-dihydrochlorid, 1,6-Di-(N₁,N₁'-alpha-methyl-.beta.-phenyldiguanido-N₅,N₅')-hexan-dihydrochlorid, 1,6-Di-(N₁,N₁'-p-nitrophenyldiguanido-N₅,N₅')hexan-dihydrochlorid, omega:omega-Di-(N₁,N₁'-phenyldiguanido-N₅,N₅)-di-n-propylether-dihydrochlorid, omega:omega'-Di-(N₁,N₁'p-chlorophenyldiguanido-N₅,N₅ )-di-n-propylether-tetrahydrochiorid, 1,6-Di-(N₁,N₁'-2,4-dichlorophenyldiguanido-N₅,N₅')hexan-tetrahydrochlorid, 1,6-Di-(N₁,N₁'-p-methylphenyldiguanido- N₅,N₅')hexan-dihydrochlorid, 1,6-Di-(N₁,N₁'-2,4,5-trichlorophenyldiguanido-N₅,N₅' )hexan-tetrahydrochlorid, 1,6-Di-[N₁,N₁'-alpha-(p-chlorophenyl) ethyldiguanido-N₅,N₅']hexan-dihydrochlorid, omega:omega-Di-(N₁,N₁'-p-chlorophenyldiguanido-N₅,N₅')mxylene-dihydrochlorid, 1,12-Di-(N₁,N₁'-p-chlorophenyldiguanido-N₅,N₅') dodecan-dihydrochlorid, 1,10-Di-(N₁,N₁'-phenyldiguanido- N₅,N₅')-decan-tetrahydrochlorid, 1,12-Di-(N₁,N₁'phenyldiguanido- N₅,N₅') dodecan-tetrahydrochlorid, 1,6-Di-(N₁,N₁'-o-chlorophenyldiguanido- N₅,N₅') hexan-dihydrochlorid, 1,6-Di-(N₁,N₁'-o-chlorophenyldiguanido- N₅,N₅') hexan-tetrahydrochlorid, Ethylen-bis-(1-tolyl biguanid), Ethylen-bis-(p-tolyl biguanide), Ethylen-bis-(3,5-dimethylphenylbiguanid), Ethylen-bis-(p-tert-amylphenylbiguanid), Ethylenbis-(nonylphenylbiguanid), Ethylen-bis-(phenylbiguanid), Ethylen-bis-(N-butylphenylbiguanid), Ethylen-bis (2,5-diethoxyphenylbiguanid), Ethylen-bis (2,4-dimethylphenyl biguanid), Ethylen-bis (o-diphenylbiguanid), Ethylen-bis (mixed amyl naphthylbiguanid), N-Butyl-ethylen-bis-(phenylbiguanid), Trimethylen-bis-(o-tolylbiguanid), N-Butyl-trimethylebis-(phenyl biguanide) und die entsprechenden Salze wie Acetate, Gluconate, Hydrochloride, Hydrobromide, Citrate, Bisulfite, Fluoride, Polymaleate, N-Cocosalkylsarcosinate, Phosphite, Hypophosphite, Perfluorooctanoate, Silicate, Sorbate, Salicylate, Maleate, Tartrate, Fumarate, Ethylendiamintetraacetate, Iminodiacetate. Cinnamate, Thiocyanate, Arginate, Pyromellitate, Tetracarboxybutyrate, Benzoate, Glutarate, Monofluorphosphate, Perfluorpropionate sowie beliebige Mischungen davon. Weiterhin eignen sich halogenierte Xylol- und Kresolderivate, wie p-Chlormetakresol oder p-Chlor-meta-xylol, sowie natürliche antimikrobielle Wirkstoffe pflanzlicher Herkunft (z.B. aus Gewürzen oder Kräutern), tierischer sowie mikrobieller Herkunft. Vorzugsweise können antimikrobiell wirkende oberflächenaktive quatemäre Verbindungen, ein natürlicher antimikrobieller Wirkstoff pflanzlicher Herkunft und/oder ein natürlicher antimikrobieller Wirkstoff tierischer Herkunft, äußerst bevorzugt mindestens ein natürlicher antimikrobieller Wirkstoff pflanzlicher Herkunft aus der Gruppe, umfassend Coffein, Theobromin und Theophyllin sowie etherische Öle wie Eugenol, Thymol und Geraniol, und/ oder mindestens ein natürlicher antimikrobieller Wirkstoff tierischer Herkunft aus der Gruppe, umfassend Enzyme wie Eiweiß aus Milch, Lysozym und Lactoperoxidase, und/ oder mindestens eine antimikrobiell wirkende oberflächenaktive quaternäre Verbindung mit einer Ammonium-, Sulfonium-, Phosphonium-, lodonium- oder Arsoniumgruppe, Peroxoverbindungen und Chlorverbindungen eingesetzt werden. Auch Stoffe mikrobieller Herkunft, sogenannte Bakteriozine, können eingesetzt werden.

Die als antimikrobielle Wirkstoffe geeigneten quatemären Ammoniumverbindungen (QAV) weisen die allgemeine Formel (R¹)(R²(R³)(R⁴) N⁺ X⁻ auf, in der R¹ bis R⁴ gleiche oder verschiedene C₁-C₂₂-Alkylreste, C₇-C₂₈-Aralkylreste oder heterozyklische Reste, wobei zwei oder im Falle einer aromatischen Einbindung wie im Pyridin sogar drei Reste gemeinsam mit dem Stickstoffatom den Heterozyklus, z.B. eine Pyridinium- oder Imidazoliniumverbindung, bilden, darstellen und X⁻ Halogenidionen, Sulfationen, Hydroxidionen oder ähnliche Anionen sind. Für eine optimale antimikrobielle Wirkung weist vorzugsweise wenigstens einer der Reste eine Kettenlänge von 8 bis 18, insbesondere12 bis 16, C-Atomen auf.

QAV sind durch Umsetzung tertiärer Amine mit Alkylierungsmitteln, wie z.B. Methylchlorid, Benzylchlorid, Dimethylsulfat, Dodecylbromid, aber auch Ethylenoxid herstellbar. Die Alkylierung von tertiären Aminen mit einem langen Alkyl-Rest und zwei Methyl-Gruppen gelingt besonders leicht, auch die Quatemierung von tertiären Aminen mit zwei langen Resten und einer Methyl-Gruppe kann mit Hilfe von Methylchlorid unter milden Bedingungen durchgeführt werden. Amine, die über drei lange Alkyl-Reste oder Hydroxysubstituierte Alkyl-Reste verfügen, sind wenig reaktiv und werden bevorzugt mit Dimethylsulfat quatemiert.

Geeignete QAV sind beispielweise Benzalkoniumchlorid (N-Alkyl-N,N-dimethyl-benzylammoniumchlorid, CAS No. 8001-54-5), Benzalkon B (*m,p*-Dichiorbenzyl-dimethyl-C12-alkylammoniumchlorid, CAS No. 58390-78-6), Benzoxoniumchlorid (Benzyl-dodecyl-bis-(2-hydroxyethyl)-ammonium-chlorid), Cetrimoniumbromid (N-Hexadecyl-N,N-trimethylammoniumbromid, CAS No. 57-09-0), Benzetoniumchlorid (N,N-Dimethyl-N-[2-[2-[*p* (1,1,3,3-tetramethylbutyl)-pheno-xy]ethoxy]ethyl]-benzylammoniumchlorid, CAS No. 121-54-0), Dialkyldimethylammonium-chloride wie Di-*n*-decyl-dimethyl-ammoniumchlorid (CAS No. 7173-51-5-5), Didecyldi-methylammoniumbromid (CAS No. 2390-68-3), Dioctyldimethyl-ammoniumchloric, 1-Cetylpyridiniumchlorid (CAS No.123-03-5) und Thiazoliniodid (CAS No. 15764-48-1) sowie deren Mischungen. Besonders bevorzugte QAV sind die Benzalkoniumchloride mit C₈-C₁₈-Alkylresten, insbesondere C₁₂-C₁₄-Aklyl-benzyl-dimethylammoniumchlorid.

Benzalkoniumhalogenide und/ oder substituierte Benzalkoniumhalogenide sind beispielsweise kommerziell erhältlich als Barquat® ex Lonza, Marquat® ex Mason, Variquat® ex Witco/ Sherex und Hyamine® ex Lonza, sowie Bardac® ex Lonza. Weitere kommerziell erhältliche antimikrobielle Wirkstoffe sind N(3-Chlorallyl)-hexaminiumchlorid wie Dowicide® und Dowicil® ex Dow, Benzethoniumchlorid wie Hyamine® 1622 ex Rohm & Haas, Methylbenzethoniumchlorid wie Hyamine® 10X ex Rohm & Haas, Cetylpyridiniumchlorid wie Cepacolchlorid ex Merrell Labs.

Die antimikrobiellen Wirkstoffe werden in Mengen von 0,0001 Gew.-% bis 1 Gew.-%, bevorzugt von 0,001 Gew.-% bis 0,8 Gew.-%, besonders bevorzugt von 0,005 Gew.-% bis 0,3 Gew.-% und insbesondere von 0,01 bis 0,2 Gew.-% eingesetzt.

Ferner können die oben zur Beschichtung der Kapseln genannten kationischen Verbindungen ebenfalls enthalten sein.

Die kosmetischen Mittel können neben den Mikro- und/oder Nanokapseln mit kationischer Oberflächenladung alle sonstigen in derartigen Mitteln üblicherweise enthaltenen Komponenten enthalten.

Hautpflegemittel enthalten üblicherweise lipophile Bestandteile. Die erfindungsgemäß enthalienen Kapseln sowie ggf. weitere Komponenten werden üblicherweise zunächst in diese lipophlilen Bestandteile eingearbeitet, in dem man sie mit geeigneten lipophilen Komponenten und Emutgatoren nach bekannten Verfahren in eine feinteilige Dispersion überführt. Ein solches Verfahren zur Herstellung feindisperser Dispersionen ist z.B. aus DE 43 37 030 A1 bekannt. Die nach dem dort beschriebenen Phaseninversions-Verfahren (PIT-Verfahren) erhaltenen Dispersionen weisen eine niedrige Viskosität und eine sehr niedrige Teilchengröße von weniger als 500 nm, bevorzugt 100 - 200 nm, auf und eignen sich vorzüglich als Träger für lipidlösliche Wirkstoffe in kosmetischen und dermatologischen Zubereitungen zur Behandlung und Pflege der Haut.

Als lipophile Bestandteile sind insbesondere Wachse zu nennen. Hierbei eignen sich natürliche und künstlich gewonnene Wachse, die bei 20°C fest und knetbar, grob- bis feinkristallin sind und erst oberhalb von etwa 40°C ohne Zersetzung in einen fließfähigen, niedrigerviskosen Zustand übergehen.

Art und Herkunft dieser Wachse unterliegen im Rahmen der vorliegenden Erfindung keinerlei Beschränkungen. Die Wachse können demgemäß aus jeder der drei Gruppen gewählt werden, in die die Wachse üblicherweise eingeteilt sind. Bei diesen Gruppen handelt es sich um
1) natürliche Wachse und zwar
   (a) pflanzliche Wachse, die unterteilt werden in rezente Wachse wie Candelilla-, Carnauba-, Japan-, Espartogras-, Ouricoury-Wachs und in fossile Wachse Montanwachs, usw.;
   (b) tierische Wachse wie Bienen-, Schellack-Wachs, Walrat, Lanolin (Wollwachs), Bürzelfett usw.;
   (c) Mineralwachse (Erdötwachse), wie Ceresin, Ozokerit (Erdwachs), Petroleum, Paraffin- und Mikrowachse,
2) chemisch veränderte Wachse, z.B. die aus Rohmontanwachs hergestellten oxidierten Produkte,
3) synthetische Wachse, auch Kunstwachse genannt, z.B. aus den nach dem Fischer-Tropsch-Verfahren gewonnenen Paraffinen, die durch Luftoxidation, selektiver Lösungsmittelbehandlung, Veresterung, Verseifung usw. in eine Reihe von Hartwachsen überführt werden. Auch Polyethylenwachse, die teils durch Hochdruckpolymerisation von Ethylen teils durch Crackung aus dem hochmolekularen Niederdruckpolyethylen hergestellt werden, sowie entsprechende oxidierte Produkte, zählen zu den synthetischen Wachsen.

Weiterhin eignen sich als Wachse auch Fettsäureester, von ein- oder mehrwertigen Alkoholen, die aufgrund ihrer Konsistenz und ihres Schmelzbereichs ein wachsartiges Verhalten zeigen. Solche Produkte sind z.B. die Ester gesättigter, linearer C₁₂-C₂₂-Fettsäuren und gesättigter, linearer C₁₂-C₂₂-Fettalkohole, z.B. Cetylpalmitat, Stearylstearat oder Cetylbehenat, Triglyceride gesättigter linearer C₁₂-C₂₂-Fettsäuren und gehärtete pflanzliche und tierische Fette, wie z.B. gehärtetes Palmfett, gehärteter Talg oder gehärtetes Rizinusöl.

Die Herstellung von Hautpflegemitteln und der Haarpflegemittel, sofern sie in Form von Wasser-in-Öl- bzw. ÖI-in-Wasser-Emulsionen vorliegen, erfolgt vorzugsweise in der Weise, daß man die lipophilen Komponenten sowie ggf. weitere wasserunlösliche Komponenten vermischt und diese dann entweder als solche oder in Form einer wässerigen Dispersion bei einer Temperatur unterhalb des Schmelzpunktes der lipophilen Teilchen in die übrige Zubereitung einmischt.

Zur Herstellung der Emulsionen werden üblicherweise Emulgatoren eingesetzt. Die Art und Menge der benötigten Emulgatoren wird nach dein HLB-Wert der lipophilen Komponenten und der Menge der zu emulgierenden lipophilen Komponenten, Wachse und Wirkstoffe, in an sich bekannter Weise gewählt.

Besonders gut geeignete Emulgatoren sind z. B. nichtionogene Verbindungen mit einer lipophilen Alkyl- oder Acylgruppe und einer hydrophilen Polyol- oder Polyethergruppe. Beispiele für geeignete Emulgatoren sind z.B. Ethylenoxidanlagerungsprodukte an Fettalkohole mit 16 - 22 Kohlenstoffatomen, an Fettsäuremonoglyceride oder an Sorbitanmonoester von Fettsäuren mit 16 - 22 Kohlenstoffatomen. Besonders gut geeignete Emulgatoren weisen einen HLB-Wert von 8 - 18 auf. Dabei wird als HLB-Wert eine Größe verstanden, die sich aus der Beziehung HLB = 0,2 x (100-L) ergibt. Darin ist L der Gewichtsanteil der lipophilen Gruppen, d.h. der Fettalkyl- oder Fettacylgruppen in Prozent in den nichtionogenen Emulgatoren.

Zur Herstellung der Mittel kann die Mitverwendung eines lipophilen Coemulgators von Vorteil sein. Dabei handelt es sich um polare Lipide mit einer oder zwei C₁₂-C₂₇Alkyl- oder Acylgruppen und einer hydrophilen Gruppe, deren Größe nicht ausreicht, um das Molekül wasserlöslich zu machen, z.B. die Hydroxylgruppe, eine Dihydroxyethylgruppe oder eine Polyhydroxyalkoxygruppe mit 3 - 6 Kohlenstoffatomen und 2 - 5 Hydroxylgruppen. Solche polaren Lipide werden oft auch als "lipophile Coemulgatoren" bezeichnet. Geeignete Beispiele für solche Lipide sind z.B. Cetyl- und Stearylalkohol, 1,2-Dodecandiol, Glycerinmonocethylether, Glycerinmonostearat, Stearylmonoglucosid, Sorbitanmonopalmitat oder Methylglucosid-dioleat.

Die z.B. nach dem PIT-Verfahren hergestellten, feinteiligen Dispersionen wirkstoffhaltiger Wachspartikel lassen sich in beliebige wässerige Zubereitungen einarbeiten. Als wässerige Zubereitungen sind dabei Cremes, Emulsionen und Dispersionen, Gel und Lotionen zu verstehen, deren äußere Phase wässerig ist. Besonders bevorzugt sind flüssige Öl-in-Wasser-Emulsionen oder Öl-in-Wasser-Cremes. Auch gemischte Emulsionstypen, z.B. vom Typ Wasser-in-ÖI-in-Wasser eignen sich zur Einbringung der mit anorganischen Verbindungen und gegebenenfalls lipophilen Wirkstoffen beladenen Wachsdispersionen.

Solche wässerigen Zubereitungen können alle üblichen Komponenten solcher Zubereitungen in den üblichen Mengen enthalten. So können z. B. übliche kosmetische Öfkomponenten in emulgierter oder mikroemulgierter Form, Emulgatoren, Verdickungsmittel, wasserlösliche Wirkstoffe, Proteine oder Proteinderivate, Complexbildner, Puffersalze, Konservierungsstoffe, Pigmente und Farbstoffe in üblichen Mengen enthalten sein.

Die erfindungsgemäßen Haarpflegemittel können alle in solchen Zubereitungen bekannten Wirk-, Zusatz- und Hilfsstoffe enthalten.

Weitere Wirk-, Hilfs- und Zusatzstoffe, die in Haarpflegemitteln enthalten sein können, sind beispielsweise
- nichtionische Polymere wie beispielsweise Vinylpyrrolidon/Vinylacrylat-Copolymere, Polyvinylpyrrolidon und VinylpyrrolidonNinylacetat-Copolymere und Polysiloxane,
- kationische Polymere wie quatemisierte Celluloseether, Polysiloxane mit quatemären Gruppen, Dimethyldiallylammoniumchlorid-Polymere, Acrylamid-Dimethyldiallylammoniumchlorid-Copolymere, mit Diethylsulfat quatemierte Dimethylaminoethylmethacrylat-Vinylpyrrolidon-Copolymere, Vinylpyrrolidon-Imidazoliniummethochlorid-Copolymere und quatemierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylatltert.-Buiylaminoethylmethacrylat/2-Hydroxypropylmethacrytat-Copolymere,
- anionische Polymere wie beispielsweise Polyacrylsäuren, vemetzte Polyacrylsäuren, Vinylacetat/Crotonsäure-Copolymere, VinylpyrrolidonNinylacrylat-Copolymere, Vinylacetat/Butylmaleat/Isobomylacrylat-Copolymere, Methylvinylether/Maleinsäureanhydrid-Copolymere und Acrylsäure/Ethylacrylat/N-tert: Butylacrylamid-Terpolymere,
- Verdickungsmittel wie Agar-Agar, Guar-Gum, Alginate, Xanthan-Gum, Gummi arabicum, Karaya-Gummi, Johannisbrotkemmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quatemisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs. Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Herstellung der Haarpflegemittel kann in an sich bekannter Weise durch Vermischen der inhaltsstoffe erfolgen, wobei auch Vorgemische einzelner Komponenten eingesetzt werden können.

### Beispiele

### Beispiel 1: Effekte auf Textilien

Weichspülerformulierungen wurden mit 0,2 % bis 5 % von Duftstoff enthaltenden Mikrokapseln, die analog zu dem im EP 0839 902 (BASF) beschriebenen Verfahren hergestellt und in erfindungsgemäßer Weise kationisch modifiziert wurden, versetzt. Das Verhältnis Kem:Wand betrug in allen Fällen zwischen 90:10 und 75:25.

Anschließend wurden Textilien aus Baumwolle mit einer Weichspülerlösung 5 min behandelt. Das Mengenverhältnis von Weichspüler zu Trockenwäsche betrug 10,3 g / kg, das Flottenverhältnis betrug 1 : 5.

Durch sensorische Methoden konnte beim Bügeln, durch leichtes Reiben sowie bei der Behandlung im Trockner eine Duftfreisetzung im Vergleich mit der Referenzprobe detektiert werden. Der Nachweis der Substantivität der Kapseln auf dem Gewebe wurde mit Hilfe der konfokalen Mikroskopie erbracht.

Die Rezepturen der Kapseln enthaltenden Weichspüler waren die in Tabelle 1 aufgelisteten:

**Tabelle 1:**

| Rezepturen für Weichspüler, Mengenangaben in Gew.-%. | | | | |
|---|---|---|---|---|
| **Rezeptur** | **M 1** | **M 2** | **M 3** | **M 4** |
| kationisches Tensid¹ | 5,0 | 16,0 | 21,5 | 21,5 |
| Polyethylen-Dispersion² | - | - | - | 3,0 |
| MgCl₂ · 6 H₂O | 0,3 | 0,55 | 0,55 | 0,55 |
| Mikrokapseln³ mit Duftstoff⁴ | 0,2 | 0,2 | 0,2 | 0,2 |
| freies Parfüm, Farbstoff, Verdicker, Entschäumer, Wasser | Rest | Rest | Rest | Rest |

| **Rezeptur** | **M 5** | **M 6** | **M 7** | **M 8** |
|---|---|---|---|---|
| kationisches Tensid¹ | 5.0 | 16.0 | 21,5 | 21,5 |
| Polyethylen-Dispersion² | - | - | - | 3,0 |
| MgCl₂ · 6 H₂O | 0,3 | 0,55 | 0,55 | 0,55 |
| Mikrokapseln³ mit Duftstoff⁴ | 0,5 | 0,5 | 0,5 | 0,5 |
| freies Parfüm, Farbstoff, Verdicker, Entschäumer, Wasser | Rest Rest | Rest Rest | Rest Rest | Rest Rest |

| **Rezeptur** | **M 9** | **M 10** | **M 11** | **M 12** |
|---|---|---|---|---|
| kationisches Tensid¹ | 5,0 | 16,0 | 21,5 | 21,5 |
| Polyethylen-Dispersion² | - | - | - | 3,0 |
| MgCl₂ · 6 H₂O | 0,3 | 0,55 | 0,55 | 0,55 |
| Mikrokapseln³ mit Duftstoff⁴ | 1,0 | 1,0 | 1,0 | 1,0 |
| freies Parfüm, Farbstoff, Verdicker, Entschäumer, Wasser | Rest | Rest | Rest | Rest |
| | | | | |

| **Rezeptur** | **M 13** | **M 14** | **M 15** | **M 16** |
|---|---|---|---|---|
| kationisches Tensid¹ | 5,0 | 16,0 | 21,5 | 21,5 |
| Polyethylen-Dispersion² | - | - | - | 3,0 |
| MgCl₂· 6 H₂O | 0,3 | 0,55 | 0,55 | 0,55 |
| Mikrokapseln³ mit Duftstoff⁴ | 2,0 | 2,0 | 2,0 | 2,0 |
| freies Parfüm, Farbstoff, Verdicker, Entschäumer, Wasser | Rest | Rest | Rest | Rest |
| | | | | |

| **Rezeptur** | **M 17** | **M 18** | **M 19** | **M 20** |
|---|---|---|---|---|
| kationisches Tensid¹ | 5.0 | 16,0 | 21,5 | 21,5 |
| Polyethylen-Dispersion² | - | - | - | 3,0 |
| MgCl₂ · 6 H₂O | 0,3 | 0,55 | 0,55 | 0,55 |
| Mikrokapsein³ mit Duftstoff⁴ | 5,0 | 5.0 | 5,0 | 5,0 |
| freies Parfüm, Farbstoff, Verdicker, Entschäumer, Wasser | Rest | Rest | Rest | Rest |

| | | | | |
|---|---|---|---|---|
| ¹ Stepantex VL 90 A® , N-Methyl-N(2-hydroxyethyl)-N,N-(ditalgacyloxyethyl)ammoniummethosulfat, additiviert mit Stepanquat X 9124® ex Stepan Europe, 3-Talgamidopropyldimethylammoniummethosulfat, 90 % in Isopropanol | | | | |
| ² Adalin K® ex Henkel, 20gew.-%ige Dispersion in Wasser, entspricht 0,6 Gew.-% Polyethylen, aktive Substanz | | | | |
| ³ verschiedene Größenverteilungen: Mediane bei ca. 5, 20 und 40 µm (a, b, c) | | | | |
| ⁴ jeweils mit Rosenöl, Pfefferminzöl, Orangenöl (I, II, III) | | | | |

Die Substantivität wurde ebenfalls über die Wahmehmung der Intensität der Parfümöle auf den behandelten Textilien bestimmt. Als Vergleichsmittel wurden jeweils Weichspüler gewählt, deren Anteil an freiem Parfüm dem Gesamtparfümanteit (frei + verkapselt) der erfindungsgemäßen Mittel entsprach.

Die Wahrnehmung der Intensität der Parfümöle erfolgte zum einen nach 2, 6 und 9 Tagen Schranktagerung nach Ausrüstung, Schleudern und eintägigem, hängendem Trocknen. Hier zeigte sich, dass die mit erfindungsgemäßen Mitteln ausgerüsteten Textilien nach Lagerung noch eine deutlich stärkere Duftintensität aufwiesen als die gleichlang gelagerten Referenzmuster.

Zum anderen erfolgte die Wahmehmung der Parfümölintensität nach Ausrüstung, Schleudern und eintägigem, hängendem Trocknen über den Dufteindruck beim anschließenden Bügeln. Auch hier zeigten die mit erfindungsgemäßen Mitteln ausgerüsteten Textilien eine deutlich stärkere Duftintensität als die Referenzmuster.

### Beispiel 2: Effekte auf Haaren

Für alle Untersuchungen wurden braune europäische Haare mittlerer Länge verwendet. Diese Haare wurden zunächst einer Vorreinigung mit einer wäßrigen 12 Gew.-%igen Natriumlaurylsulfat-Lösung (mit Zitronensäure auf pH 3 eingestellt) unterzogen und anschließend mit einer wäßrigen 6 Gew.-%igen Wasserstoffperoxid-Lösung (mit Ammoniak auf pH 9,4 eingestellt) mediumblondiert.

### Beispiel 2.1: Aufziehen kationisch modifizierter Mikrokapseln mit ursprünglich anionischer Oberflächenladung auf Haare

### Beispiel 2.1.1 Aufziehen aus wäßriger Suspension

10 ml einer 40 Gew.-%igen wäßrigen Suspension von Rosenöl-gefüllten Mikrokapseln (negative Oberflächenladung, durchschnittlicher Durchmesser 4 bis 8 µm), welche nach dem in EP 0 839 902 beschriebenen Verfahren hergestellt wurden, wurden in 50 ml einer wäßrigen 0,25 Gew.-%igen Cetrimoniumchlorid-Lösung (Dehyquart A, kationisch) suspendiert. Dabei bildeten sich unter Umladung Kapseln mit einer positiv geladenen Oberfläche, was durch Zetapotential-Messungen gezeigt wurde. Anschließend wurde das Gemisch mit 7 Gew.-Teiten demineralisierten Wassers verdünnt und mit Zitronensäure (10 Gew.-%ig in Wasser) auf pH 5,0 eingestellt. Die, wie eingangs beschrieben, vorbehandelten Haarsträhnen wurden 15 sec. in dieses Gemisch eingetaucht und danach 1 Min unter fließendem Wasser (1l/min x Strähne, 38°C) abgespült. Anschließend wurden die Haare bei konstanter Luftfeuchtigkeit 16 Std. getrocknet.

Der Nachweis der Kapseln auf dem Haar erfolgte auf zweierlei Weise:
a) sensorisch:
   Der Geruch der behandelten Haare wurde von 6 Testpersonen zunächst als "leicht nach Rosenöl" beschrieben. Dann wurde die Haarsträhne zwischen den Fingern gerieben und der Geruch erneut beurteilt: Der Duft wurde nun als "viel frischer" und "intensiver beschrieben. Bei analoger Versuchsdurchführung wie vorstehend beschrieben, wobei jedoch der pH nicht auf 5,0. sondern auf 7,5 bzw. 3,8 eingestellt worden war, wurde eine geringere Intensität des sensorisch wahrgenommenen Dufteindruckes festgestellt.
b) optisch:
   Behandelte und unbehandelte Haare wurden mikroskopisch bei 1250facher Vergrößerung untersucht. Im Falle der behandelten Haare konnten auf der Haaroberfläche anhaftende Kapseln (3 Kapseln auf 0,1 mm Länge eines untersuchten Haarabschnitts) nachgewiesen werden.

### Beispiel 2.1.2: Aufziehen aus einer Haarspülung

Zunächst wurde aus 4 g Cetearyl Alcohol, 0,5 g Ceteareth-20, 0,16 g Cetrimoniumchlorid und 95,34 g Wasser eine Basis-Haarspülung hergestellt, welche einen pH-Wert von 4,6 aufwies. In 50 g dieser Spülung wurden 2,5 g einer Kapselsuspension eingearbeitet, welche durch Vermischen von 10 ml der in Beispiel 2.1.1 verwendeten 40 Gew.-%igen wäßrigen Suspension von Rosenöl-gefüllten Mikrokapseln mit 50ml einer 0,25 Gew.-%igen wäßrigen Dehyquart-A-Lösung erhalten worden war. 1g der so erhaltenen Rosenölkapselhaltigen Haarspülung wurde auf 1g Haar aufgetragen und 5 min einwirken gelassen. Anschließend wurden die Haare 1 min unter fließendem Wasser (1l/min x Strähne, 38°C) ausgespült und 16 Std. bei konstanter Luftfeuchtigkeit getrocknet.

Der Nachweis der Kapseln am Haar erfolgte auf zweierlei Weise:
a) sensorisch:
   Der Geruch der behandelten Haare wurde von 6 Testpersonen zunächst als "leicht nach Rosenöl duftend" beschrieben. Dann wurde die Haarsträhne zwischen den Fingern gerieben und der Geruch erneut beurteilt: Der Duft wurde nun als "viel frischer" und "intensiver" beurteilt. In analoger Weise wurde ein Dufteffekt durch Kämmen der Haarsträhne erzielt, wobei die mit Parfümöl gefüllten Kapseln, welche nach der Behandlung am Haar haften blieben, aufbrachen und ihren Duft freisetzten.
b) optisch:
   Behandelte und unbehandelte Haare wurden mikroskopisch bei 1250facher Vergrößerung untersucht. Im Falle der behandelten Haare konnten auf der Haaroberfläche anhaftende Kapseln nachgewiesen werden.

### Beispiel 2.2: Aufziehen von Mikrokapseln mit kationischer Polymeroberfläche auf Haare

### Beispiel 2.2.1 Aufziehen aus wäßriger Suspension

Nach dem in EP 0 839 902 beschriebenen Verfahren wurde unter Verwendung kationischer Monomere eine 50 Gew.-%ige wäßrige Dispersion kationischer Mikrokapseln hergestellt. Die Kapseln wurden mit Paraffinöl gefüllt. Sie wiesen einen durchschnittlichen Durchmesser von 2,2 µm auf. Anschließend wurde daraus durch Verdünnen mit Wasser eine 2 Gew.-%ige Dispersion hergestellt. Die, wie eingangs von Beispiel 2 beschrieben, vorbehandelten Haarsträhnen wurden 15 sec. in diese Lösung eingetaucht und danach 1 min unter fließendem Wasser (1l/min x Strähne, 38°C) gespült. Anschließend wurden die Haare bei konstanter Luftfeuchtigkeit 16 Std. getrocknet.

Der Nachweis der Kapseln auf der Haaroberfläche erfolgte wiederum mikroskopisch. Es konnten auf der Haaroberfläche anhaftende Kapseln nachgewiesen werden.

### Beispiel 2.2.2 Aufziehen aus einer Haarspülung

In 100 g der Basis-Haarspülung, welche in Beispiel 2.1.2 beschrieben ist, wurden 2,0 g einer 50 Gew.-%igen wäßrigen Dispersion der in Beispiel 2.2.1 beschriebenen Kapseln eingearbeitet. 1g der erhaltenen Formulierung wurde auf 1g Haar aufgetragen, und 5 min einwirken gelassen. Anschließend wurden die Haare 1 min unter fließendem Wasser (1l/min x Strähne, 38°C) ausgespült und 16 Std. bei konstanter Luftfeuchtigkeit getrocknet.

Der Nachweis der Kapseln auf der Haaroberfläche erfolgte wiederum mikroskopisch. Es konnten auf der Haaroberfläche anhaftende Kapseln nachgewiesen werden.

## Patentansprüche

1. Wasch- oder Reinigungsmittel, **dadurch gekennzeichnet, daß** es ein Kombinationsprodukt ist, das Komponenten für die Textilreinigung und Komponenten für die Textilpflege in Form von Mikro- und/oder Nanokapseln, deren Oberfläche kationische Ladungen aufweisen, enthält.

2. Mittel nach Anspruch 1, **dadurch gekennzeichnet, daß** die Kapseln aus einem Kern und einem den Kern umgebenden Wandmaterial aufgebaut sind.

3. Mittel nach Anspruch 1, **dadurch gekennzeichnet, daß** die Kapseln kompakt sind oder der Kern keine aktive Komponente enthält.

4. Mittel nach einem der Ansprüche 1 bis 3, **dadurch gekennzeichnet, daß** die Kapseln eine Teilchengröße von 10 nm bis 1000 µm aufweist.

5. Mittel nach Anspruch 4, **dadurch gekennzeichnet, daß** die Kapseln eine Teilchengröße von 1 bis 60 µm, vorzugsweise 1,5 bis 40 µm und insbesondere 2 bis 30 µm aufweisen.

6. Mittel nach einem der Ansprüche 1 bis 5, **dadurch gekennzeichnet, daß** das Massenverhältnis Kern: Wand zwischen 99,9:0,1 und 2:1, vorzugsweise zwischen 95:5 und 70:30 und insbesondere zwischen 90:10 und 75:25 liegt.

7. Mittel nach einem der Ansprüche 1 bis 6, **dadurch gekennzeichnet, daß** die Freisetzung des Kernmaterials mittels thermischer Energie oder durch Druck erfolgt.

8. Mittel nach einem der Ansprüche 1 bis 6, **dadurch gekennzeichnet, daß** die Freisetzung des Kernmaterials mittels pH-Sprung oder Osmose erfolgt.

9. Mittel nach einem der Ansprüche 1 bis 8, **dadurch gekennzeichnet, daß** die Freisetzung des Kernmaterials beim oder nach dem Aufbringen auf die Oberfläche, insbesondere auf das Textil erfolgt.

10. Mittel nach einem der Ansprüche 1 bis 9, **dadurch gekennzeichnet, daß** die Oberfläche der Kapseln ganz oder teilweise aus einem kationischen Polymer besteht oder mit einer kationischen Verbindung beschichtet ist.

11. Mittel nach Anspruch 10, **dadurch gekennzeichnet, daß** die kationischen Verbindungen ausgewählt sind aus quaternären Ammoniumverbindungen der Formeln (I) und (II), 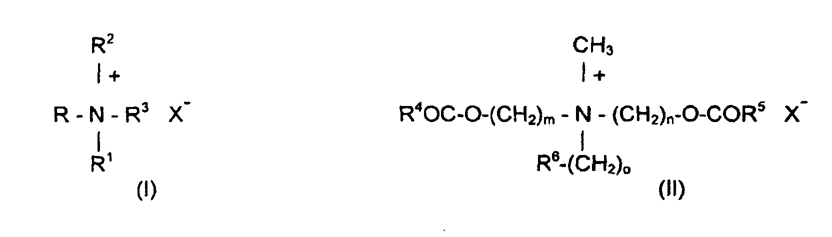 wobei R und R¹ für einen acyclischen Alkylrest mit 12 bis 24 Kohlenstoffatomen, R² für einen gesättigten C₁-C₄ Alkyl- oder -Hydroxyalkylrest steht, R³ entweder gleich R, R¹ oder R² oder ein aromatischer Rest ist und COR⁴ und COR⁵ jeweils für einen aliphatischen Acylrest mit 12 bis 22 Kohlenstoffatomen mit 0, 1, 2 oder 3 Doppelbindungen steht sowie R⁶ für H oder OH steht, wobei m, n und o jeweils unabhängig voneinander den Wert 1, 2 oder 3 haben können und X entweder ein Halogenid-, Methosulfat- , Methophosphat- oder Phosphation ist, quaternären Imidazoliniumverbindungen der Formel (IV) 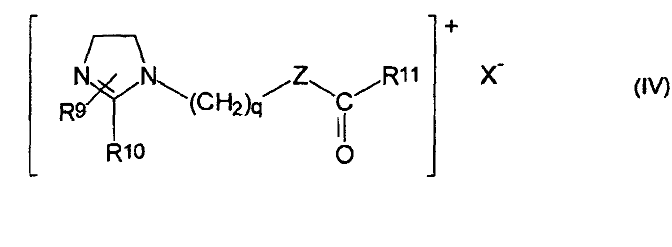 wobei R⁹ für H oder einen gesättigten Alkylrest mit 1 bis 4 Kohlenstoffatomen, R¹⁰ und R¹¹ unabhängig voneinander jeweils für einen aliphatischen, gesättigten oder ungesättigten Alkylrest mit 12 bis 18 Kohlenstoffatomen, R¹⁰ alternativ auch für O(CO)R²⁰ stehen kann, wobei R²⁰ einen aliphatischen, gesättigten oder ungesättigten Alkylrest mit 12 bis 18 Kohlenstoffatomen bedeutet, und Z eine NH-Gruppe oder Sauerstoff bedeutet und X⁻ ein Anion ist. q kann ganzzahlige Werte zwischen 1 und 4 annehmen,
quaternären Verbindungen der Formel (V) 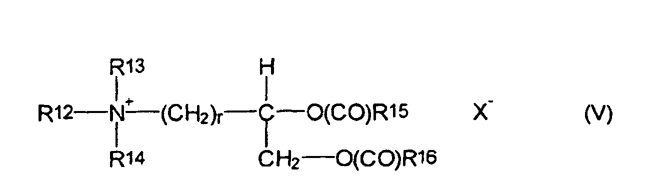 wobei R¹², R¹³ und R¹⁴ unabhängig voneinander für eine C₁₋₄ Alkyl-, Alkenyl- oder Hydroxyalkylgruppe steht, R¹⁵ und R¹⁶ jeweils unabhängig ausgewählt eine C₈₋₂₈-Alkylgruppe darstellt und r eine Zahl zwischen 0 und 5 ist,
kurzkettigen, wasserlöslichen, quaternären Ammoniumverbindungen, wie Trihydroxyethylmethylammonium-methosulfat oder die Alkyltrimethylammoniumchloride, Dialkyldimethylammoniumchloriden und Trialkylmethylammoniumchloriden, protonierten Alkylaminverbindungen, quaternisierten Proteinhydrolysate, Polyquaternium-Polymeren, kationischen quaternären Zuckerderivaten, Copolymeren von PVP und Dimethylaminomethacrylat, Copolymeren von Vinylimidazol und Vinylpyrrolidon, Aminosilicon-potymeren und -copolymeren, polyquaternierten Polymeren, kationischen Biopolymeren auf Chitinbasis, kationischen Silikonölen, Alkylamidoaminen, quaternären Esterverbindungen.

12. Mittel nach einem der Ansprüche 1 bis 11, **dadurch gekennzeichnet, daß** das Kapselmaterial ausgewählt ist aus natürlichen oder synthetischen Polymeren, insbesondere polymeren Polysacchariden, wie Agarose oder Cellutose, Stärke, Chitin, Chitosan, Proteinen, wie Gelatine, Gummi arabicum, Ethylcellulose, Methylcellulose, Carboxymethylethylcellulose, Hydroxyethylcellulose, Celluloseacetaten, Polyamiden, Polycyanacrylaten, Polylactiden, Polyglycoliden, Polyanilin, Polypyrrol, Polyvinylpyrrolidon, Polystyrol, Polyvinylalkohol, Copolymeren aus Polystyrol und Maleinsäureanhydrid, Epoxidharzen, Polyethyleniminen, Copolymeren aus Styrol und Methylmethacrylat, Polyacrylaten und Polymethacrylaten, Polycarbonaten, Polyestern, Silikonen, Gemischen aus Gelatine und Wasserglas, Gelatine und Polyphosphat, Celluloseacetat und Phthalat, Gelatine und Copolymeren aus Maleinsäureanhydrid und Methylvinylether, Celluloseacetatbutyrat sowie beliebigen Gemischen der voranstehenden, die kationische Gruppen aufweisen können.

13. Mittel nach einem der Ansprüche 1 bis 8, **dadurch gekenrueichnet, daß** als Kernmaterialien Duftstoffe, pflegende Komponenten, Vitamine, wie Vitamin E (α-Tocopherol), Panthenol (Provitamin B5) und Betakarotin (Provitamin A), Antischuppenmittel, UV-Schutzmittel, Emollients (kosmetische Öle), Silikonöle, Conditioner, Glycerin, Polymere für Festigungseffekte beim Haar, kationische Polymere, Komponenten zur Textilausrüstung und -veredelung, wie imprägniermittel, Appretur, Avivagemittel, Komponenten für die Pflegeleichtausrüstung, Griffvariatoren und Soil-Release-Ausrüstung, Antistatika, antimikrobielle und fungizide Mittel eingesetzt werden.

14. Mittel nach einem der Ansprüche 1-13, **dadurch gekennzeichnet, daß** es ein Mittel zur Textilbehandlung, insbesondere ein Universalwaschmittel, Feinwaschmittel, Textilvorbehandlungsmittel, Fleckenbehandlungsmittel, Nachbehandlungsmittel, wie ein Weichspüler, zur Polsterreinigung oder Teppichreinigung ist.

15. Mittel nach einem der Ansprüche 1-14, **dadurch gekennzeichnet, daß** es in flüssiger bis gelförmiger oder fester Form vorliegt.

## Claims

1. Washing or cleaning composition, **characterized in that** it is a combination product which comprises components for the cleaning of textiles and components for the care of textiles in the form of microcapsules and/or nanocapsules, the surfaces of which have cationic charges.

2. Composition according to Claim 1, **characterized in that** the capsules are constructed from a core and a wall material surrounding the core.

3. Composition according to Claim 1, **characterized in that** the capsules are compact or the core comprises no active component.

4. Composition according to one of Claims 1 to 3, **characterized in that** the capsules have a particle size of from 10 nm to 1000 µm.

5. Composition according to Claim 4, **characterized in that** the capsules have a particle size of from 1 to 60 µm, preferably 1.5 to 40 µm and in particular 2 to 30 µm.

6. Composition according to one of Claims 1 to 5, **characterized in that** the core:wall mass ratio is between 99.9:0.1 and 2:1, preferably between 95:5 and 70:30 and in particular between 90:10 and 75:25.

7. Composition according to one of Claims 1 to 6, **characterized in that** the core material is released by means of thermal energy or as a result of pressure.

8. Composition according to one of Claims 1 to 6, **characterized in that** the core material is released by means of a pH jump or osmosis.

9. Composition according to one of Claims 1 to 8, **characterized in that** the core material is released during or following application to the surface, in particular to the textile.

10. Composition according to one of Claims 1 to 9, **characterized in that** the surface of the capsules consists completely or partially of a cationic polymer or is coated with a cationic compound.

11. Composition according to Claim 10, **characterized in that** the cationic compounds are chosen from quaternary ammonium compounds of the formulae (I) and (II) , 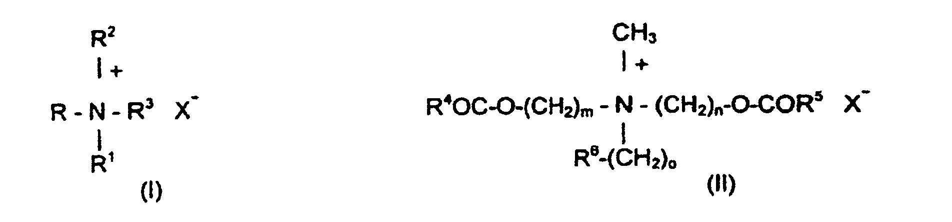 where R and R¹ are an acyclic alkyl radical having 12 to 24 carbon atoms, R² is a saturated C₁-C₄-alkyl or -hydroxyalkyl radical, R³ is either the same as R, R¹ or R² or is an aromatic radical, and COR⁴ and COR⁵ are in each case an aliphatic acyl radical having 12 to 22 carbon atoms with 0, 1, 2 or 3 double bonds, and R⁶ is H or OH, where m, n and o, in each case independently of one another, can have the value 1, 2 or 3, and X is either a halide, methosulphate, methophosphate or phosphate ion,
quaternary imidazolinium compounds of the formula (IV) 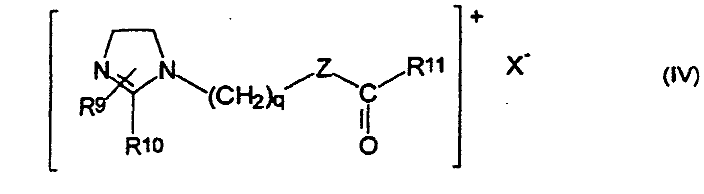 where R⁹ is H or a saturated alkyl radical having 1 to 4 carbon atoms, R¹⁰ and R¹¹, independently of each other, can in each case be an aliphatic, saturated or unsaturated alkyl radical having 12 to 18 carbon atoms, R¹⁰ alternatively may also be O (CO) R²⁰, where R²⁰ is an aliphatic, saturated or unsaturated alkyl radical having 12 to 18 carbon atoms, and Z is an NH group or oxygen and X⁻ is an anion. q can assume integer values between 1 and 4,
quaternary compounds of the formula (V) 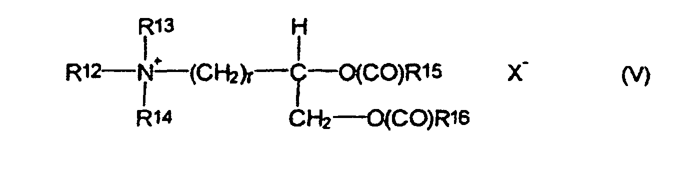 where R¹², R¹³ and R¹⁴, independently of each other, is a C₁₋₄-alkyl, -alkenyl or -hydroxyalkyl group, R¹⁵ and R¹⁶, in each case chosen independently, is a C₈₋₂₈-alkyl group and r is a number between 0 and 5,
short-chain, water-soluble, quaternary ammonium compounds, such as trihydroxyethylmethylammonium methosulphate or the alkyltrimethylammonium chlorides, dialkyldimethylammonium chlorides and trialkylmethylammonium chlorides, protonated alkylamine compounds, quaternized protein hydrolysates, polyquaternium polymers, cationic quaternary sugar derivatives, copolymers of PVP and dimethylaminomethacrylate, copolymers of vinylimidazole and vinylpyrrolidone, aminosilicone polymers and copolymers, polyquaternized polymers, cationic biopolymers based on chitin, cationic silicone oils, alkylamidoamines, quaternary ester compounds.

12. Composition according to one of Claims 1 to 11, **characterized in that** the capsule material is chosen from natural or synthetic polymers, in particular polymeric polysaccharides, such as agarose or cellulose, starch, chitin, chitosan, proteins, such as gelatin, gum arabic, ethylcellulose, methylcellulose, carboxymethylethylcellulose, hydroxyethylcellulose, cellulose acetates, polyamides, polycyanoacrylates, polylactides, polyglycolides, polyaniline, polypyrrol, polyvinylpyrrolidone, polystyrene, polyvinyl alcohol, copolymers of polystyrene and maleic anhydride, epoxy resins, polyethyleneimines, copolymers of styrene and methyl methacrylate, polyacrylates and polymethacrylates, polycarbonates, polyesters, silicones, mixtures of gelatin and waterglass, gelatin and polyphosphate, cellulose acetate and phthalate, gelatin and copolymers of maleic anhydride and methyl vinyl ether, cellulose acetate butyrate, and any mixtures of the above, which may have cationic groups.

13. Composition according to one of Claims 1 to 8, **characterized in that** the core materials used are fragrances, care components, vitamins, such as vitamin E (α-tocopherol), panthenol (provitamin B5) and betacarotine (provitamin A), antidandruff agents, UV protectants, emollients (cosmetic oils), silicone oils, conditioners, glycerol, polymers for setting effects in hair, cationic polymers, components for finishing and refining of textiles, such as impregnating agents, finish, softeners, components for easy-care finishing, hand modifiers and soil-release finishing, antistats, antimicrobial and fungicidal agents.

14. Composition according to one of Claims 1-13, **characterized in that** it is a composition for the treatment of textiles, in particular a universal detergent, light-duty detergent, textile pretreatment agent, stain treatment agent, after-treatment agent, such as a fabric softener, for the cleaning of upholstery or carpet cleaning.

15. Composition according to one of Claims 1-14, **characterized in that** it is in liquid to gel-like or solid form.

## Revendications

1. Agent de lavage ou de nettoyage, **caractérisé en ce qu'**il s'agit d'un produit de combinaison qui contient des composants pour le nettoyage des textiles et des composants pour le soin des textiles sous la forme de microcapsules et/ou de nanocapsules dont la surface présente des charges cationiques.

2. Agent selon la revendication 1, **caractérisé en ce que** les capsules sont composées d'un noyau et d'une matière de paroi entourant le noyau.

3. Agent selon la revendication 1, **caractérisé en ce que** les capsules sont compactes ou le noyau ne contient pas de composant actif.

4. Agent selon l'une quelconque des revendications 1 à 3, **caractérisé en ce que** les capsules présentent une granulométrie de 10 nm à 1000 µm.

5. Agent selon la revendication 4, **caractérisé en ce que** les capsules présentent une granulométrie de 1 à 60 µm, de préférence de 1,5 à 40 µm et en particulier de 2 à 30 µm.

6. Agent selon l'une quelconque des revendications 1 à 5, **caractérisé en ce que** le rapport de masse noyau : paroi se situe entre 99,9 : 0,1 et 2 : 1, de préférence entre 95 : 5 et 70 : 30 et en particulier entre 90 : 10 et 75 : 25.

7. Agent selon l'une quelconque des revendications 1 à 6, **caractérisé en ce que** la libération de la matière du noyau a lieu à l'aide d'énergie thermique ou sous l'influence de la pression.

8. Agent selon l'une quelconque des revendications 1 à 6, **caractérisé en ce que** la libération de la matière du noyau a lieu à l'aide d'un saut de pH ou par osmose.

9. Agent selon l'une quelconque des revendications 1 à 8, **caractérisé en ce que** la libération de la matière du noyau a lieu lors de l'application sur la surface, en particulier sur le textile, ou après ladite application.

10. Agent selon l'une quelconque des revendications 1 à 9, **caractérisé en ce que** la surface des capsules est constituée en tout ou en partie d'un polymère cationique ou bien est enduite d'un composé cationique.

11. Agent selon la revendication 10, **caractérisé en ce que** les composés cationiques sont choisis parmi des composés d'ammonium quaternaires répondant aux formules (I) et (II), 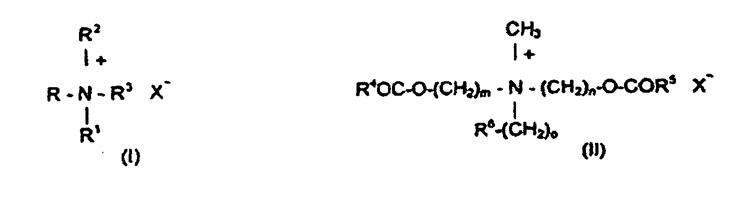 dans lesquelles R et R¹ représentent un radical alkyle acyclique contenant de 12 à 24 atomes de carbone, R² représente un radical alkyle en C₁-C₄ ou hydroxyalkyle en C₁-C₄ saturé, R³ est égal soit à R, R¹ ou R², ou bien représente un radical aromatique, et COR⁴ et COR⁵ représentent respectivement un radical acyle aliphatique contenant de 12 à 22 atomes de carbone et comprenant 0, 1, 2 ou 3 liaisons doubles, et R⁶ représente un atome d'hydrogène ou un groupe OH, m, n et o pouvant prendre, respectivement, indépendamment l'un de l'autre, la valeur 1, 2 ou 3 et X représente soit un ion d'halogénure, soit un ion de méthosulfate, soit un ion de méthophosphate, soit un ion de phosphate, parmi des composés d'imidazolinium quaternaires répondant à la formule (IV) 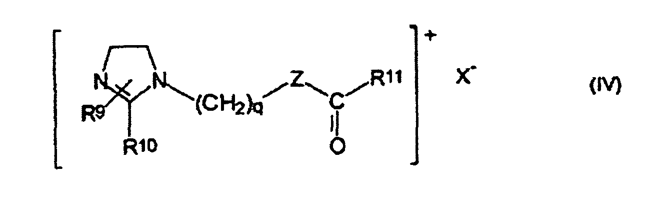 dans laquelle R⁹ représente un atome d'hydrogène ou un radical alkyle saturé contenant de 1 à 4 atomes de carbone, R¹⁰ et R¹¹ représentent indépendamment l'un de l'autre, respectivement un radical alkyle aliphatique, saturé ou insaturé contenant de 12 à 18 atomes de carbone, R¹⁰ peut également représenter en variante un groupe O(CO)R²⁰ dans lequel R²⁰ représente un radical alkyle aliphatique, saturé ou insaturé contenant de 12 à 18 atomes de carbone, et Z représente un groupe NH ou un atome d'oxygène et X⁻ représente un anion, q pouvant prendre des valeurs de nombres entiers entre 1 et 4,
parmi des composés quaternaires répondant à la formule (V) 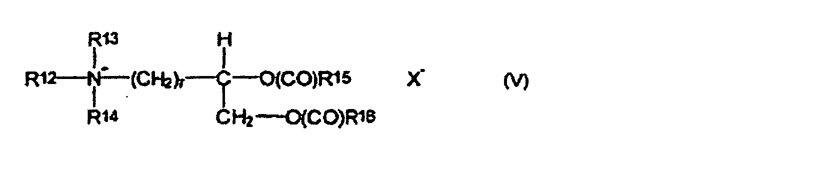 dans laquelle R¹², R¹³ et R¹⁴ représentent, indépendamment l'un de l'autre, un groupe alkyle en C₁-C₄, un groupe alcényle ou un groupe hydroxyalkyle, R¹⁵ et R¹⁶ représentent respectivement un groupe alkyle en C₈-C₂₈, choisi de manière indépendante, et r représente un nombre entre 0 et 5,
parmi des composés d'ammonium quaternaires, solubles dans l'eau, à courtes chaînes, tels que le méthosulfate de trihydroxyéthylméthylammonium ou les chlorures d'alkyltriméthylammonium, les chlorures de dialkyldiméthylammonium et les chlorures de trialkylméthyl-ammonium, des composés d'alkylamines protonés, des hydrolysats protéiniques quaternisés, des polymères de polyquaternium, des dérivés quaternaires cationiques du sucre, des copolymères de polyvinylpyrrolidone et de diméthylaminométhacrylate, des copolymères de vinylimidazole et de vinylpyrrolidone, des polymères et des copolymères d'aminosilicium, des polymères polyquaternisés, des biopolymères cationiques à base de chitine, des huiles de silicone cationiques, des alkylamidoamines, des composés d'esters quaternaires.

12. Agent selon l'une quelconque des revendications 1 à 11, **caractérisé en ce que** la matière de la capsule est choisie parmi le groupe comprenant des polymères naturels ou synthétiques, en particulier des polysaccharides polymères tels que l'agarose ou la cellulose, l'amidon, la chitine, le chitosane, des protéines telles que la gélatine, la gomme arabique, l'éthylcellulose, la méthylcellulose, la carboxyméthyléthylcellulose, l'hydroxyéthylcellulose, des acétates de cellulose, des polyamides, des polycyanacrylates, des polylactides, des polyglycolides, la polyaniline, le polypyrrole, la polyvinylpyrrolidone, le polystyrène, l'alcool polyvinylique, des copolymères de polystyrène et d'anhydride de l'acide maléique, des résines époxydes, des polyéthylèneimines, des copolymères de styrène et de méthacrylate de méthyle, des polyacrylates et des polyméthacrylates, des polycarbonates, des polyesters, des silicones, des mélanges de gélatine et de verre soluble, de gélatine et de polyphosphate, d'acétate de cellulose et de phtalate, de gélatine et de copolymères d'anhydride de l'acide maléique et d'éther méthylvinylique, de l'acétobutyrate de cellulose, ainsi que n'importe quels mélanges des éléments précités qui peuvent présenter des groupes cationiques.

13. Agent selon l'une quelconque des revendications 1 à 8, **caractérisé en ce que** l'on met en oeuvre, à titre de matières du noyau, des matières odoriférantes, des composants pour les soins, des vitamines telles que la vitamine E ( -tocophérol), le panthénol (provitamine B5) et le bêtacarotène (provitamine A), des agents antipelliculaires, des agents de protection contre les ultraviolets, des émollients (huiles cosmétiques), des huiles de silicone, des agents de conditionnement, du glycérol, des polymères pour des effets de permanente dans les cheveux, des polymères cationiques, des composants pour le gommage et le finissage des textiles, tels que des agents d'imprégnation, des agents de apprêtage, des agents d'avivage, des composants pour conférer un entretien facile sans repassage, des agents pour faire varier le toucher et des agents conférant un apprêt de type « antisa)issure », des agents antistatiques, des agents antimicrobiens et des agents fongicides.

14. Agent selon l'une quelconque des revendications 1 à 13, **caractérisé en ce qu'**il s'agit d'un agent pour le traitement des textiles, en particulier un agent de lavage universel, un agent de lavage pour le linge délicat, un agent de prétraitement des textiles, un agent de traitement des taches, un agent de traitement ultérieur tel qu'un adoucissant, un agent pour le nettoyage des coussins ou un agent de nettoyage pour les tapis.

15. Agent selon l'une quelconque des revendications 1 à 14, **caractérisé en ce qu'**il est présent sous une forme allant d'une forme liquide à une forme de gel ou sous forme solide.
